# EUROPEAN PATENT APPLICATION

(11) **EP 4 825 884 A1**
(43) Date of publication of application: **21.10.2026**
(21) Application number: 26156396.9
(22) Date of filing: 04.02.2026
(51) Int. Cl.: C08F 8/00, G01N 33/53

(54) **COPOLYMER, CONJUGATE PREPARATION KIT, EXOSOME COLLECTION KIT, METHOD FOR PRODUCING CONJUGATE FOR COLLECTING EXOSOME, EXOSOME-COLLECTING METHOD, CONJUGATE FOR COLLECTING EXOSOME, AUTOMATED DEVICE, AND ANALYSIS SYSTEM**

(30) Priority: 18.04.2025 JP 2025068872
(71) Applicant: National Institute for Materials Science, Tsukuba-shi, Ibaraki 305-0047 (JP); SPHinX Inc., Tsukuba-shi, Ibaraki 3050047 (JP)
(72) Inventor: TOLBA, Ahmed Nabil Ahmed Elsayed, Ibaraki, 3050047 (JP); EBARA, Mitsuhiro, Ibaraki, 3050047 (JP); SASAKI, Makoto, Ibaraki, 3050047 (JP)
(74) Representative: Seemann & Partner Patentanwälte mbB

(57) **Abstract**

[Object] To provide a copolymer that solves at least one of problems in conventional technology relating to exosome collection.

[Solution] A copolymer includes a unit 1 represented by a following formula 1 and a unit 2 represented by a following formula 2, wherein when total units included in the copolymer is 100 mol%, the content of the unit 2 is 2.5 mol% or more and 9.5 mol% or less, 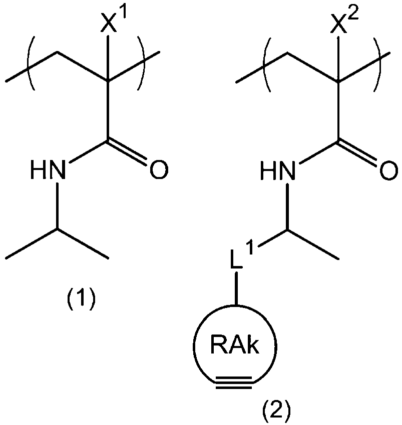 (in the formula 1, X¹ represents a hydrogen atom or a linear or branched alkyl group having 1 to 6 carbon atoms, and in the formula 2, X² represents a hydrogen atom or a linear or branched alkyl group having 1 to 6 carbon atoms, L¹ represents a divalent linking group, and RAk represents a cyclic alkynyl group having a monocyclic structure or a fused ring structure).

## Description

### Technical Field

The present disclosure relates to a copolymer, a conjugate preparation kit, an exosome collection kit, a method for producing a conjugate for exosome collection, a conjugate for exosome collection, an exosome-collecting method, an automated device, and an analysis system.

### Background Art

"Temperature-responsive polymers" that cause phase transition depending on a change in the temperature of the solution and change the physical properties are known. As such a temperature-responsive polymer, Patent Literature 1 describes a polymer of an "isopropylacrylamide derivative including an azido group or an alkyne group".

### Citation List

### Patent Literature

Patent Literature 1: Japanese Unexamined Patent Application Publication No. 2012-201634

### Summary of Invention

### Technical problem

Exosomes are one of extracellular vesicles, have a variety of sizes, and are generally defined as vesicles having a membrane structure with a diameter of 30 to 200 nm. It is known that exosomes play an important role in transmitting information between cells and include a variety of biological molecules such as a protein, a lipid, and a ribonucleic acid (RNA) therein. The group of these biological molecules reflects the conditions and properties of cells (host cells) from which exosomes are derived, and exosomes are attracting attention as a diagnosis marker and a therapeutic target for diseases. It has been reported that exosome-specific molecular patterns correlate to disease progression and prognosis, particularly, in cancer, nervous disease, immunological disease, and so on, and it is expected that analyzing this will lead to early diagnosis of disease and personalized medicine. In addition, an exosome itself can be used as a therapeutic drug delivery system (DDS), and its application range is extremely wide.

In exosome analysis, it is believed that it is important to collect an exosome in its original shape and structure. Three main reasons thereof are as follows. One is for accurate analysis. The structures and contents of exosomes are recognized to reflect the conditions of the cells from which the exosomes are derived and characteristics of disease. Accordingly, in a disrupted exosome, reliability of diagnosis and analysis may decrease. Another is for widening the possibility of therapeutic use. When an exosome is used as a DDS, the completeness of the membrane structure is recognized to highly influence the encapsulation efficiency of a drug and the transportation efficiency to a target cell. In a disrupted exosome, there is a possibility that the therapeutic effect is not sufficiently exhibited. Another is from the viewpoint of ensuring the purity. In a sample contaminated with another extracellular vesicle or a protein complex, there is a possibility of increasing the risk that analysis results and therapeutic effects will be incorrect. Collection of a pure exosome is indispensable for obtaining highly reliable and reproducible results.

However, it has been believed that it is difficult to collect an exosome in its original shape and structure. Three main reasons thereof are as follows. One is lacking uniformity in size. The size of exosomes is broad, from 30 to 200 nm, and therefore it is difficult to separate the exosomes from other extracellular vesicles and protein complexes based on only the size. That is, there is a possibility that structures other than exosomes can be mixed by ultracentrifugation or filtering. Another is a risk of physical disruption. Even if the collection by ultracentrifugation is possible, the exosomes may be disrupted by the centrifugal force during the collection and the high shear force in the separation process, and there is a risk of losing the membrane structure and the internal molecule. Consequently, there is a possibility that the functionality and specific characteristics of the collected exosomes are harmed. This can be a fatal problem depending on the purpose of analysis or use. Another is a problem of non-specific adsorption. Exosomes include a lot of lipids and proteins on the membrane surface and thereby may non-specifically adsorb to the filter or the reagent during collection operation. Consequently, the yield decreases, and the specificity may be harmed.

The present disclosure solves at least one of the problems in the above-described conventional technology relating to exosome collection.

### Solution to Problem

One of the copolymers of the present disclosure is a copolymer including a unit 1 represented by a formula 1 described later and a unit 2 represented by a formula 2 described later. When the total units included in the copolymer is 100 mol%, the content of the unit 2 is 2.5 mol% or more and 9.5 mol% or less.

One of the methods for producing a conjugate for exosome collection of the present disclosure is a method for producing a conjugate for exosome collection comprising forming a linker-ligand molecule complex by binding a linker represented by a formula 4 described later and a ligand molecule selected from the group consisting of a protein, an antibody, a peptide, and derivatives thereof, and generating a conjugate by reacting the linker-ligand molecule complex and a copolymer including a unit 1 represented by the formula 1 described later and a unit 2 represented by the formula 2 described later, wherein when the total units included in the copolymer is 100 mol%, the content of the unit 2 is 2.5 mol% or more and 9.5 mol% or less.

One of the exosome-collecting methods of the present disclosure is an exosome-collecting method comprising forming a linker-ligand molecule complex by binding a linker represented by the formula 4 described later and a ligand molecule selected from the group consisting of a protein, an antibody, a peptide, and derivatives thereof; forming a conjugate by reacting the linker-ligand molecule complex and a copolymer including a unit 1 represented by the formula 1 described later and a unit 2 represented by the formula 2 described later, wherein when the total units included in the copolymer is 100 mol%, the content of the unit 2 is 2.5 mol% or more and 9.5 mol% or less, bringing the conjugate into contact with an exosome in a solution to immobilize at least a part of the exosome to the conjugate through the ligand molecule, and heating the solution to a temperature not lower than an aggregation temperature set in advance based on the lower critical solution temperature of the copolymer to aggregate the exosome together with the conjugate.

A first conjugate for exosome collection of the present disclosure is a conjugate for exosome collection including a unit 1 represented by the formula 1 described later and a unit 6 represented by the formula 6 described later.

One of the conjugate preparation kits for exosome collection of the present disclosure is a conjugate preparation kit for exosome collection, comprising a copolymer including a unit 1 represented by the formula 1 described later and a unit 2 represented by the formula 2 described later, and a linker represented by the formula 4 described later.

One of the exosome collection kits of the present disclosure is an exosome collection kit comprising a copolymer including a unit 1 represented by the formula 1 described later and a unit 2 represented by the formula 2 described later, a linker represented by the formula 4 described later, and a ligand molecule selected from the group consisting of a protein, an antibody, a peptide, and derivatives thereof, wherein the ligand molecule is a molecule targeting a marker that is present in outer membrane of an exosome.

One of the exosome-collecting methods of the present disclosure is an exosome-collecting method comprising forming a linker-ligand molecule complex by binding a linker represented by the formula 4 described later and a ligand molecule selected from the group consisting of a protein, an antibody, a peptide, and derivatives thereof and targeting a marker that is present in outer membrane of an exosome, forming a conjugate by reacting the linker-ligand molecule complex and a copolymer including a unit 1 represented by the formula 1 described later and a unit 2 represented by the formula 2 described later, bringing the conjugate into contact with an exosome in a solution to immobilize at least a part of the exosome to the conjugate through the ligand molecule, and heating the solution to a temperature not lower than an aggregation temperature set in advance based on the lower critical solution temperature of the copolymer to aggregate the exosome together with the conjugate.

### Advantageous Effects of Invention

According to the present disclosure, at least one of the problems in the above-described conventional technology relating to exosome collection can be solved.

### Brief Description of Drawings

Fig. 1 is a flow chart illustrating a procedure of an exosome-collecting method.
Fig. 2 is an image sequence showing a manner of separating cells from mouse liver in the procedure of extracting mesenchymal stem cells (MSCs).
Fig. 3 is an image sequence showing a manner of separating MSCs only by removing stem cells in the procedure of extracting mesenchymal stem cells (MSCs).
Fig. 4 is TEM images of exosomes collected by ultracentrifugation.
Fig. 5 is TEM images of exosomes collected using a conjugate prepared by binding a ligand molecule (anti-CD81) to a copolymer.
Fig. 6 is images showing the results of SDS-PAGE.
Fig. 7 is a graph showing the measurement results of protein concentrations with a microvolume spectrophotometer (NanoDrop).
Fig. 8 is a graph showing the verification results of the amount of collected exosomes.
Fig. 9 is a graph showing the expression amounts of CD63 (surface marker of exosome) in collected matters.
Fig. 10 is graphs showing the particle diameter measurement results of exosomes by DLS.
Fig. 11 is a graph showing the average diameter of particles.
Fig. 12 is a graph showing the verification results of the amount of collected exosomes when a conjugate prepared using Tim-4, which is a protein that binds to a phosphatidylserine group in a calcium ion-dependent manner, is used as a ligand molecule.
Fig. 13 is photographs showing the TEM observation results of exosomes separated by acting a chelating agent (EDTA) on a conjugate collected by heating and aggregation.

### Description of Embodiments

In the present specification, a numerical range represented by A to B includes A as the lower limit and B as the upper limit unless otherwise specified.
[1] A copolymer comprising a unit 1 represented by a following formula 1 and a unit 2 represented by a following formula 2, wherein when total units included in the copolymer is 100 mol%, the content of the unit 2 is 2.5 mol% or more and 9.5 mol% or less, 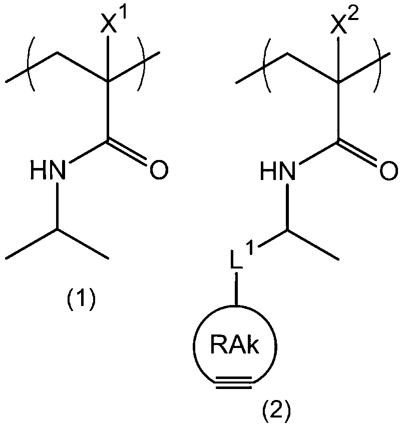 (in the formula 1, X¹ represents a hydrogen atom or a linear or branched alkyl group having 1 to 6 carbon atoms, and in the formula 2, X² represents a hydrogen atom or a linear or branched alkyl group having 1 to 6 carbon atoms, L¹ represents a divalent linking group, and RAk represents a cyclic alkynyl group having a monocyclic structure or a fused ring structure).
[2] The copolymer according to [1], wherein the copolymer is for an exosome collection kit.
[3] A conjugate preparation kit comprising the copolymer according to [1] or [2] and a linker represented by a following formula 4: 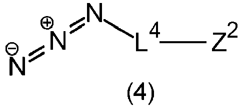 (in the formula 4, L⁴ represents a divalent linking group, and Z² represents a reactive group that can bind to a ligand molecule selected from the group consisting of a protein, an antibody, a peptide, and derivatives thereof).
[4] The conjugate preparation kit according to [3], wherein the ligand molecule is a molecule targeting a marker that is present in outer membrane of an exosome.
[5] The conjugate preparation kit according to [4], wherein the marker is a phosphatidylserine group.
[6] The conjugate preparation kit according to [4] or [5], wherein the ligand molecule is a protein that binds to the marker metal-ion-dependently.
[7] An exosome collection kit comprising the copolymer according to [1] or [2], a linker represented by a following formula 4, and a ligand molecule selected from the group consisting of a protein, an antibody, a peptide, and derivatives thereof, 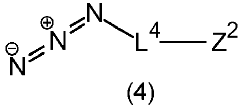 (in the formula 4, L⁴ represents a divalent linking group, and Z² represents a reactive group that can bind to the ligand molecule).
[8] The exosome collection kit according to [7], wherein the ligand molecule is a molecule targeting a marker that is present in outer membrane of an exosome.
[9] The exosome collection kit according to [8], wherein the marker is a phosphatidylserine group.
[10] The exosome collection kit according to [8] or [9], wherein the ligand molecule is a protein that binds to the marker metal-ion-dependently.
[11] A method for producing a conjugate for exosome collection, the method comprising forming a linker-ligand molecule complex by binding a linker represented by a following formula 4 and a ligand molecule selected from the group consisting of a protein, an antibody, a peptide, and derivatives thereof, and generating a conjugate by reacting the linker-ligand molecule complex and a copolymer including a unit 1 represented by a following formula 1 and a unit 2 represented by a following formula 2, wherein when total units included in the copolymer is 100 mol%, the content of the unit 2 is 2.5 mol% or more and 9.5 mol% or less, 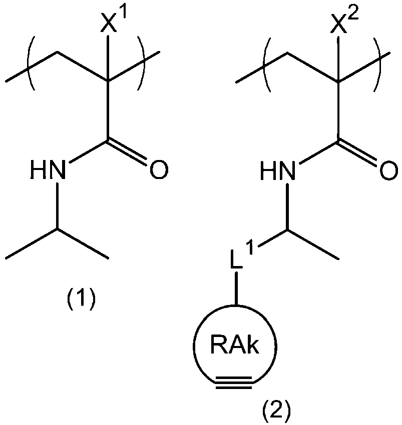 (in the formula 1, X¹ represents a hydrogen atom or a linear or branched alkyl group having 1 to 6 carbon atoms, and in the formula 2, X² represents a hydrogen atom or a linear or branched alkyl group having 1 to 6 carbon atoms, L¹ represents a divalent linking group, and RAk represents a cyclic alkynyl group having a monocyclic structure or a fused ring structure), 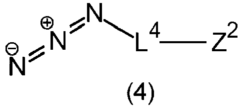 (in the formula 4, L⁴ represents a divalent linking group, and Z² represents a reactive group that can bind to the ligand molecule).
[12] An exosome-collecting method comprising forming a linker-ligand molecule complex by binding a linker represented by a following formula 4 and a ligand molecule selected from the group consisting of a protein, an antibody, a peptide, and derivatives thereof; forming a conjugate by reacting the linker-ligand molecule complex and a copolymer including a unit 1 represented by a following formula 1 and a unit 2 represented by a following formula 2, wherein when total units included in the copolymer is 100 mol%, the content of the unit 2 is 2.5 mol% or more and 9.5 mol% or less; bringing the conjugate into contact with an exosome in a solution to immobilize at least a part of the exosome to the conjugate through the ligand molecule; and heating the solution to a temperature not lower than the aggregation temperature set in advance based on the lower critical solution temperature of the copolymer to aggregate the exosome together with the conjugate, 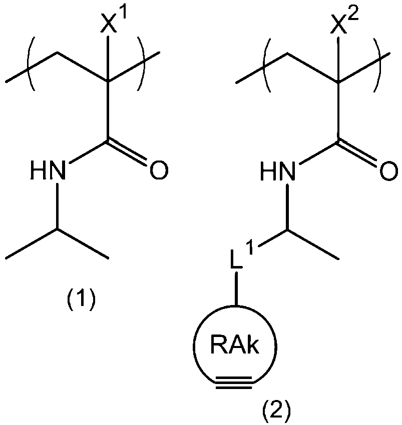 (in the formula 1, X¹ represents a hydrogen atom or a linear or branched alkyl group having 1 to 6 carbon atoms, in the formula 2, X² represents a hydrogen atom or a linear or branched alkyl group having 1 to 6 carbon atoms, L¹ represents a divalent linking group, and RAk represents a cyclic alkynyl group having a monocyclic structure or a fused ring structure), 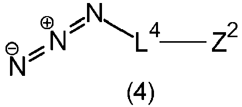 (in the formula 4, L⁴ represents a divalent linking group, and Z² represents a reactive group that can bind to the ligand molecule).
[13] The exosome-collecting method according to [12], wherein the ligand molecule is a molecule targeting a marker that is present in outer membrane of an exosome.
[14] The exosome-collecting method according to [13], wherein the marker is a phosphatidylserine group.
[15] The exosome-collecting method according to [13] or [14], wherein the ligand molecule is a protein that binds to the marker metal-ion-dependently.
[16] A conjugate for exosome collection, comprising a unit 1 represented by a following formula 1 and a unit 6 represented by a following formula 6: 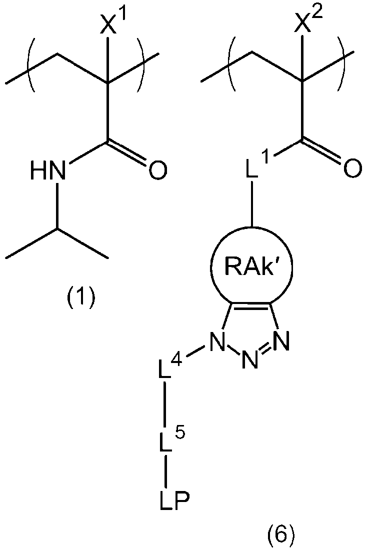 (in the formula 1, X¹ represents a hydrogen atom or a linear or branched alkyl group having 1 to 6 carbon atoms, in the formula 6, X² represents a hydrogen atom or a linear or branched alkyl group having 1 to 6 carbon atoms, L¹, L⁴, and L⁵ are the same or different divalent linking groups, RAk' represents a residue of a cyclic alkynyl group that has a monocyclic structure or a fused ring structure and is incorporated into a triazole ring formed by binding the cyclic alkynyl group and an azido group, and LP represents a residue of a ligand molecule selected from the group consisting of a protein, an antibody, a peptide, and derivatives thereof).
[17] The conjugate for exosome collection according to [16], wherein the ligand molecule is a molecule targeting a marker that is present in outer membrane of an exosome.
[18] The conjugate for exosome collection according to [17], wherein the marker is a phosphatidylserine group.
[19] The conjugate for exosome collection according to [17], wherein the ligand molecule is a protein that binds to the marker metal-ion-dependently.
[20] A conjugate preparation kit for exosome collection, the kit comprising a copolymer including a unit 1 represented by a following formula 1 and a unit 2 represented by a following formula 2, and a linker represented by a following formula 4: 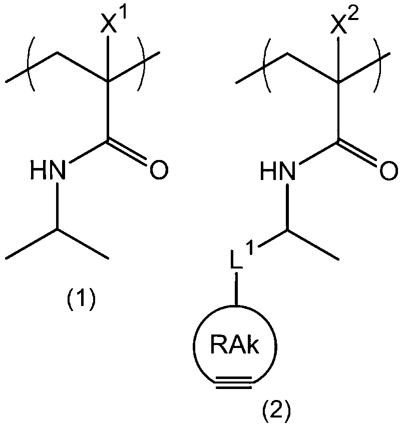 (in the formula 1, X¹ represents a hydrogen atom or a linear or branched alkyl group having 1 to 6 carbon atoms, and in the formula 2, X² represents a hydrogen atom or a linear or branched alkyl group having 1 to 6 carbon atoms, L¹ represents a divalent linking group, and RAk represents a cyclic alkynyl group having a monocyclic structure or a fused ring structure), 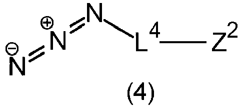 (in the formula 4, L⁴ represents a divalent linking group, and Z² represents a reactive group that can bind to a ligand molecule selected from the group consisting of a protein, an antibody, a peptide, and derivatives thereof and targeting a marker present in outer membrane of an exosome).
[21] The conjugate preparation kit according to [20], wherein the marker is a phosphatidylserine group.
[22] The conjugate preparation kit according to [20] or [21], wherein the ligand molecule is a protein that binds to the marker metal-ion-dependently.
[23] An exosome collection kit comprising a copolymer including a unit 1 represented by a following formula 1 and a unit 2 represented by a following formula 2, a linker represented by a following formula 4, and a ligand molecule selected from the group consisting of a protein, an antibody, a peptide, and derivatives thereof, wherein the ligand molecule is a molecule targeting a marker that is present in outer membrane of an exosome, 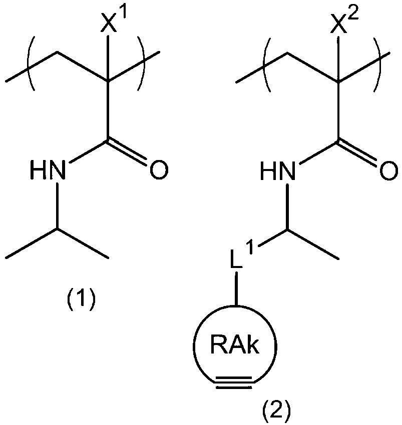 (in the formula 1, X¹ represents a hydrogen atom or a linear or branched alkyl group having 1 to 6 carbon atoms, and in the formula 2, X² represents a hydrogen atom or a linear or branched alkyl group having 1 to 6 carbon atoms, L¹ represents a divalent linking group, and RAk represents a cyclic alkynyl group having a monocyclic structure or a fused ring structure), 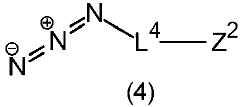 (in the formula 4, L⁴ represents a divalent linking group, and Z² represents a reactive group that can bind to the ligand molecule).
[24] The exosome collection kit according to [23], wherein the marker is a phosphatidylserine group.
[25] The exosome collection kit according to [23] or [24], wherein the ligand molecule is a protein that binds to the marker metal-ion-dependently.
[26] An exosome-collecting method comprising: forming a linker-ligand molecule complex by binding a linker represented by a following formula 4 and a ligand molecule selected from the group consisting of a protein, an antibody, a peptide, and derivatives thereof and targeting a marker that is present in outer membrane of an exosome; forming a conjugate by reacting the linker-ligand molecule complex and a copolymer including a unit 1 represented by a following formula 1 and a unit 2 represented by a following formula 2; bringing the conjugate into contact with an exosome in a solution to immobilize at least a part of the exosome to the conjugate through the ligand molecule; and heating the solution to a temperature not lower than an aggregation temperature set in advance based on the lower critical solution temperature of the copolymer to aggregate the exosome together with the conjugate, 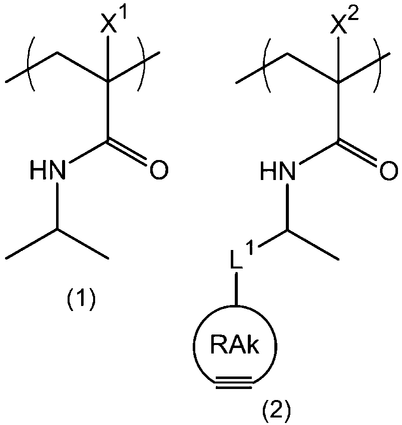 (in the formula 1, X¹ represents a hydrogen atom or a linear or branched alkyl group having 1 to 6 carbon atoms, and in the formula 2, X² represents a hydrogen atom or a linear or branched alkyl group having 1 to 6 carbon atoms, L¹ represents a divalent linking group, and RAk represents a cyclic alkynyl group having a monocyclic structure or a fused ring structure), 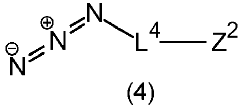 (in the formula 4, L⁴ represents a divalent linking group, and Z² represents a reactive group that can bind to the ligand molecule).
[27] The exosome-collecting method according to [26], wherein the marker is a phosphatidylserine group.
[28] The exosome-collecting method according to [26] or [27], wherein the ligand molecule is a protein that binds to the marker metal-ion-dependently.
[29] The exosome-collecting method according to [15] or [28], further comprising acting a chelating agent on the aggregated conjugate to separate the conjugate and the exosome.
[30] An automated device using the exosome collection kit according to any one of [7] to [9] and [23] to [25].
[31] An analysis system that can implement the exosome-collecting method according to [13] or [14].

### (Exosome-collecting method)

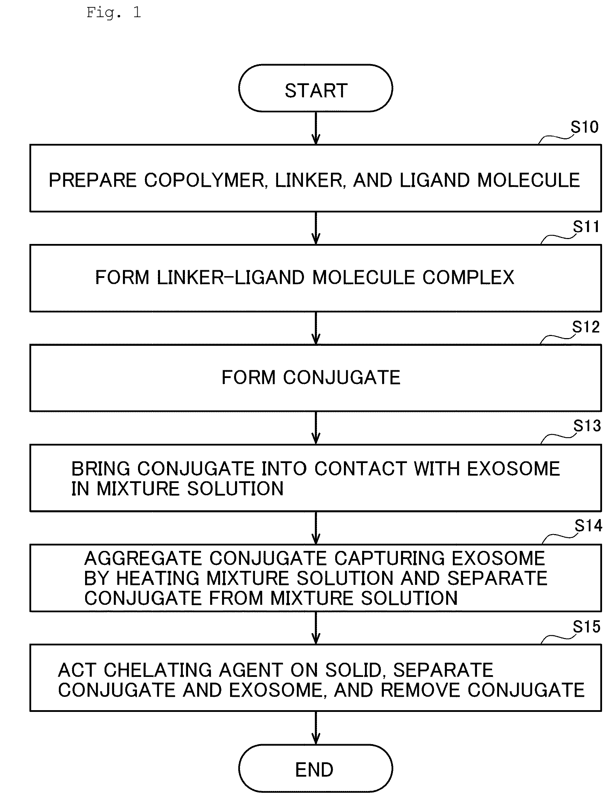

The exosome-collecting method of the present disclosure uses a conjugate of a specific copolymer and a linker-ligand molecule complex that are bound using a strain-promoted azide-alkyne cycloaddition reaction (SPAAC Reaction). Fig. 1 is a flow chart illustrating a procedure of an embodiment of the exosome-collecting method.

In the exosome-collecting method, a copolymer, a linker, and a ligand molecule are prepared in Step S10. "Preparing" can include synthesis of each component, purchase of commercially available products of each component (including addition of necessary preparation), and a combination thereof. For example, the "preparation" also includes purchase of an exosome collection kit that is supplied as a package including a copolymer, a linker, and a ligand molecule and preparing it into a condition for use. The detail of the exosome collection kit will be described later.

The copolymer used in Step S10 includes a unit 1 represented by the following formula 1 and a unit 2 represented by the following formula 2. The basic structure of this copolymer has been developed by the present inventors and is disclosed in, for example, International Publication No. WO 2022-219967. In the copolymer that is used in the exosome-collecting method of the present disclosure, in other words, in the copolymer for the exosome collection kit, when the total units included in the copolymer is 100 mol%, the content of the unit 2 is preferably 2.5 mol% or more and 9.5 mol% or less. When the content of the unit 2 is within the above numerical range, the exosome-concentrating efficiency is more improved. The relationship between the content of the unit 2 and the exosome-concentrating efficiency was only recognized when the inventors conceived and began research into using copolymers for exosome collection. In order to solve this challenge, the inventors have carried out a new study and thereby found the above numerical range. First, the structure and the synthesis method of the copolymer will be described in detail.

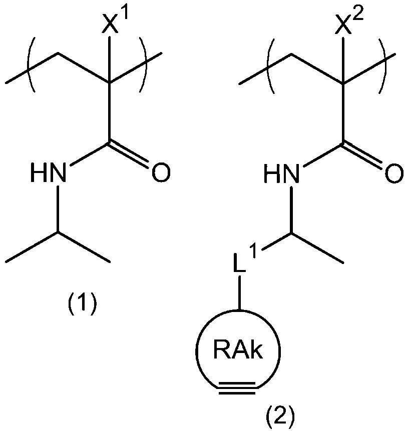

The copolymer at least includes units represented by the above formula 1 and formula 2. Here, in the formula 1, X¹ represents a hydrogen atom or a linear or branched alkyl group having 1 to 6 carbon atoms, and in the formula 2, X² represents a hydrogen atom or a linear or branched alkyl group having 1 to 6 carbon atoms, L¹ represents a divalent linking group, and RAk represents a cyclic alkynyl group having a monocyclic structure or a fused ring structure.

The unit 1 means that a polymer composed of the unit 1 alone exhibits temperature-responsiveness with a lower critical solution temperature (LCST) of 32°C relative to water. A copolymer has temperature responsiveness by containing the unit 1.

The content of the unit 1 included in the copolymer is preferably 1 to 97.5 mol% when the total repeating units is defined as 100 mol%. From the viewpoint of enhancing the capturing efficiency of an exosome, the content is more preferably 20 to 95 mol%, further preferably 70 to 95 mol%, and particularly preferably 80 to 90 mol%.

In the formula 1, X¹ represents a hydrogen atom or a linear or branched alkyl group having 1 to 6 carbon atoms, and from the viewpoint of copolymers having more sensitive temperature-responsiveness, X¹ is preferably a hydrogen atom or a linear alkyl group having 1 to 4 carbon atoms and more preferably a hydrogen atom or a methyl group.

The unit 1 is not particularly limited, but is preferably a unit based on a monomer represented by the following formula 1'. That is, the copolymer can be synthesized by copolymerization using a monomer represented by the formula 1':

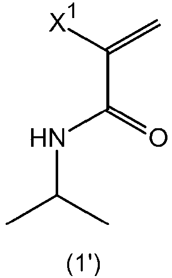

In the formula 1', X¹ is synonymous with X¹ in the formula 1, and preferable forms thereof are also the same. The monomers represented by the formula 1' may be synthesized by, for example, a known method such as a method described in Example described below or may be a commercially available product.

Regarding the above-described formula 2 again, the unit 2 constituting the copolymer includes one cyclic alkynyl group. This cyclic alkynyl group can form a fused ring structure including a triazole ring formed by [3+2] cycloaddition reaction with an azido group possessed by a linker described later. A ligand molecule can be immobilized to the copolymer through a linker by binding the linker to the ligand molecule in advance. That is, a conjugate of a ligand molecule and a copolymer can be synthesized. The synthesized conjugate aggregates and precipitates at a temperature of LCST or more due to the temperature responsiveness derived from the copolymer. Using this, an exosome as a target molecule can be concentrated.

In the copolymer that is used for exosome collection, the content of the unit 2 is preferably 1 mol% or more and 15 mol% or less when the total repeating units in the copolymer is defined as 100 mol%, and from the viewpoint of the exosome collection efficiency, the content is preferably 2.5 mol% or more and 9.5 mol% or less, 3.0 mol% or more and 9.5 mol% or less, 3.5 mol% or more and 9.5 mol% or less, 3.8 mol% or more and 9.5 mol% or less, 4.1 mol% or more and 9.5 mol% or less, or 6.0 mol% or more and 9.5 mol% or less.

In the formula 2, X² represents a hydrogen atom or a linear or branched alkyl group having 1 to 6 carbon atoms, L¹ represents a divalent linking group, and RAk represents a cyclic alkynyl group having a monocyclic structure or a fused ring structure (monovalent group including one alkynylene group in a ring structure) having, as a basic skeleton, a ring structure formed of 3 or more covalently bonded carbon atoms (the carbon atoms may be substituted with one or more hetero atoms). In the present specification, the "basic skeleton" of RAk means a ring structure including an alkynylene group. When RAk is a fused ring structure, only a ring structure including an alkynylene group is called the basic skeleton. The number of carbon atoms constituting the ring structure of the basic skeleton is preferably 4 to 20, more preferably 6 to 18, and further preferably 8 to 13. As described above, one or more of these carbon atoms may be substituted with a hetero atom such as oxygen, sulfur, and nitrogen.

In the formula 2, X² represents a hydrogen atom or a linear or branched alkyl group having 1 to 6 carbon atoms, and from the viewpoint of the copolymer having more sensitive temperature-responsiveness, X² is preferably a hydrogen atom or a linear alkyl group having 1 to 4 carbon atoms and more preferably a hydrogen atom or a methyl group.

In the formula 2, L¹ represents a divalent linking group. L¹ is not particularly limited but is preferably at least one selected from the group consisting of -O-, -S-, -C(O)-, -C(O)O-, - OC(O)O-, -NR^{A}- (R^{A} is a hydrogen atom or a monovalent substituent), -C(O)-NR^{A}-, -C(O)-S-, a linear, branched, or cyclic aliphatic hydrocarbon group having 1 to 20 carbon atoms, a monocyclic or fused-ring aromatic hydrocarbon group having 6 to 20 carbon atoms, and combinations thereof.

In particular, from the viewpoint of obtaining a copolymer having more excellent effects of the present disclosure, more preferred is one group selected from the group consisting of -O-, -S-, -C(O)-, -C(O)O-, -NH-, -C(O)-NH-, -C(O)-S-, a linear alkylene group having 1 to 10 carbon atoms, and combinations thereof.

As examples of the divalent linking group of L¹, preferred are a linear alkylene group having 1 to 10 carbon atoms itself, an oxyalkylene group in which an oxy group (=O) is bonded to a linear alkylene group having 1 to 10 carbon atoms, a structure in which linear alkylene groups having 1 to 10 carbon atoms are bonded with each other at at least one end by an ester bond, an amide bond, or a thioester bond (*-BOND-Alkylene-*: BOND represents an ester bond, an amide bond, or a thioester bond, Alkylene represents a linear alkylene group having 1 to 10 carbon atoms, and * represents the binding position), and a group composed of a combination thereof. L¹ is preferably a linear structure having 3 to 18, 4 to 15, or 5 to 10 carbon atoms in total and is further preferably -C(O)- and/or -C(O)O-.

When RAk is a monocyclic structure formed by covalently bonded carbon atoms (which may be substituted with one or more hetero atoms), the size of the ring structure of the basic skeleton (the number of carbon atoms constituting the ring) is not particularly limited, but the number of carbon atoms is preferably 8 to 13 (8- to 13-membered ring). Specifically, examples of the ring structure include cyclooctyne (number of carbon atoms: 8), cyclononyne (number of carbon atoms: 9), cyclodecyne (number of carbon atoms: 10), cycloundecyne (number of carbon atoms: 11), cyclododecyne (number of carbon atoms: 12), cyclotridecyne (number of carbon atoms: 13), and derivatives thereof. In particular, cyclooctyne or its derivative is preferable. One or more of carbon atoms constituting the above ring may be substituted with a hetero atom selected from the group consisting of a nitrogen atom, a sulfur atom, and an oxygen atom. For example, the water solubility can be adjusted by introducing an oxygen atom, the electron density can be changed by introducing a nitrogen atom, and the distortion of the ring structure can be adjusted by introducing a sulfur atom. All of them relate to the SPAAC reactivity, the method for introducing these hetero atoms and the effects to be obtained are well known, and those skilled in the art can freely implement the method. RAk is a monovalent group and is actually preferably a monovalent group having a structure obtained by removing one hydrogen atom from the above-described ring structure. This also applies to the fused ring structure.

The size of the ring relates to the progress of SPAAC reaction, i.e., reactivity. In general, an increase in the number of the carbon atoms constituting a ring often decreases the distortion, and the reaction may be slower or more selective. The reactivity can also be adjusted by introducing a substituent. Introduction of a substituent can, for example, change the electron density of a carbon-carbon triple bond, adjust the water solubility, or directly or indirectly adjust the reactivity. Examples of the monovalent substituent include a methoxy group and an electron-donating group such as an amino group. Introduction of an electron-donating group can change the electron density of a carbon-carbon triple bond and enhance the reactivity. Introduction of a hydrophilic group (a hydroxy group and an alkoxy group such as a methoxy group) enhances the solubility, and as a result, the reactivity can also be enhanced. The electron density of a carbon-carbon triple bond can be decreased by introducing an electron-withdrawing group (e.g., a halogen atom and a nitro group), and the reactivity and selectivity can be adjusted by steric hindrance through introduction of a bulky substituent (specifically, a branched monovalent hydrocarbon group having 3 to 8 carbon atoms) such as a t-Bu group.

In addition to the above, as the monovalent substituent, a carboxylic acid group and an acid group such as sulfonic acid can be adopted. The hydrophilicity and the reactivity under an acid environment can be adjusted by these groups. A monovalent substituent obtained by removing one hydrogen atom from a polyethylene glycol can function as one of hydrophilic groups. Reversely, the solubility is adjusted by introducing a hydrophobic group (such as an alkyl group and an aromatic group), and as a result, the reactivity can also be adjusted. RAk can include a derivative including one or more of the above-mentioned monovalent substituents such as an electron-donating group, a hydrophilic group, an electron-withdrawing group, a bulky substituent, an acid group, and a hydrophobic group.

The substituent that may be possessed by the ring structure can also include, in addition to the above, a carbonyl group (an oxo group (=O) bonding to a carbon atom that forms a ring structure) as a divalent substituent. In these monovalent or divalent substituents, the introduction methods and the effects induced by introduction are also well known, and those skilled in the art can easily implement the adjustment.

When RAk is a fused ring structure formed of covalently-bonded carbon atoms (which may be substituted with one or more hetero atoms), examples of the fused ring structure include a form in which one or more aromatic or aliphatic rings are fused to the cyclic alkynyl group of the above-described monocyclic structure. Examples of the fused ring of an aromatic ring such as a benzene ring include phenylcyclooctyne, dibenzocyclooctyne, dibenzoxacyclooctyne, and dibenzoazacyclooctyne. Examples of the fused ring also include the fused ring of a heterocycle such as a pyrrole ring (pyrrolocyclooctyne). Such a fused ring increases the rigidity of the cyclic alkynyl group, and selectivity and so on can be adjusted. Introduction of an aromatic ring or a heterocycle changes the electron density and can also adjust the reactivity. In addition to the above, bicyclononyne (BCN, Bicyclo[6.1.0]non-4-yne) in which a cyclooctyne ring is fused with a carbon chain can also be adopted. Bicyclononyne increases the distortion of the ring structure by ring fusion, and reactivity is improved. Also in a fused ring structure, the reactivity can be adjusted by introduction of the above-mentioned substituent. That is, a cyclic alkynyl group having a fused ring structure includes a derivative having one or more substituents described above.

More specifically, RAk is preferably a monovalent group obtained by removing one hydrogen atom from cyclooctyne or its derivative. Examples of the "derivative" in this paragraph include a derivative obtained by introducing the above-mentioned substituent into cyclooctyne, a cyclooctyne-based fused ring structure, and a derivative obtained by introducing the above-mentioned substituent into the fused ring structure. More specifically, RAk is preferably a group represented by the following formula 8 or a group obtained by introducing one or more of the above substituents into a group of the formula 8, wherein * represents a binding position with L¹.

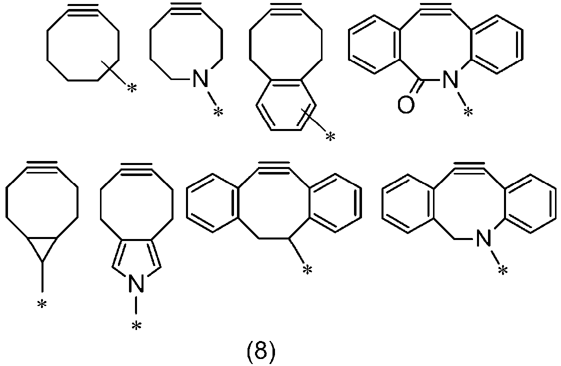

Further specifically, RAk is preferably a group represented by the following formula 81 or a group obtained by introducing one or more of the above substituents into the group of the formula 81, wherein * represents a binding position with L¹.

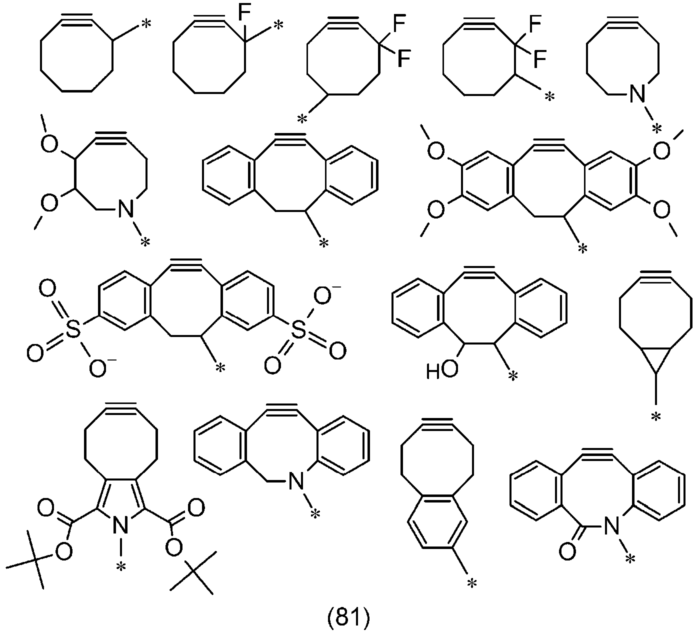

The unit 2 can be obtained by binding a precursor compound to a unit 2' represented by the following formula 2':

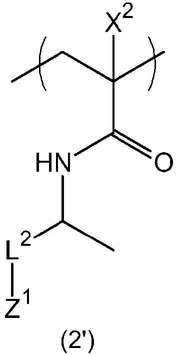

In the formula 2', X² is synonymous with X² in the formula 2, and preferable forms thereof are also the same. Examples of Z¹ include a monovalent group, such as a hydroxy group, an amino group, a carboxy group, a glycidyl group, a mercapto group, a hydroxysuccinimide ester, and maleimide, and a hydroxy group is preferable.

As the precursor compound, for example, a compound including a cyclic alkyne (e.g., cyclooctyne) that becomes a cyclic alkynyl group and a group that can react the above Z¹ may be used. As such a compound, there are commercially available products, and the compound can be synthesized by a known method. In particular, a precursor compound including a cyclic alkyne and a carboxy group is preferable, because it is easily available or easily synthesized.

When the cyclic alkyne is difluorinated cyclooctyne, as a method for synthesizing the precursor compound, the method described in schemes 1 and 2 of J. Am. Chem. Soc., 2008, 130, 34, 11486-11493 can be used. If the cyclic alkyne is dibenzoazacyclooctyne, the method described in the scheme 1 of Chemical Communications (2010), 46(1), 97-99 can be used.

As the precursor compound, BCN (bicyclo[6.1.0]nonyne), a compound obtained by adding a substituent, such as an amino group, a hydroxy group, and a carboxy group, to DBCO or the like, and maleimide and a hydroxysuccinimide ester thereof are commercially available and can be used.

From the viewpoint of obtaining the unit 2 more simply, the unit 2' is preferably a unit 5 represented by the following formula 5. In this case, as the precursor compound, typically, a precursor compound including a carboxy group and a cyclic alkyne may be used.

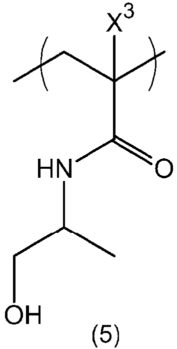

If there is an unreacted unit 5 when a copolymer is synthesized, in other words, when a copolymer includes the unit 1, the unit 2, and the unit 5, the LCST can be adjusted to the higher temperature side. The unit 5 is highly hydrophilic compared to other repeating units, the LCST can be adjusted to the higher temperature side by increasing the content of the unit 5.

In the formula 5, X³ represents a hydrogen atom or a linear or branched alkyl group having 1 to 6 carbon atoms, and from the viewpoint of copolymers having more sensitive temperature-responsiveness, X³ is preferably a hydrogen atom or a linear alkyl group having 1 to 4 carbon atoms and more preferably a hydrogen atom or a methyl group.

The content of the unit 5 in the copolymer is not particularly limited, but is preferably 0 to 10 mol% and more preferably 0 to 5 mol% when the all repeating units of a specific copolymer is 100 mol%.

The molecular weight of the copolymer is not particularly limited, but typically, the number-average molecular weight is preferably 2,000 to 100,000, more preferably 5,000 to 50,000, further preferably 10,000 to 30,000, and particularly preferably 10,000 or more and less than 20,000. When the number-average molecular weight is 5,000 or more, more excellent concentration efficiency is easily obtained (in other words, more excellent temperature-responsiveness is easily obtained). In contrast, when the molecular weight is 50,000 or less, a conjugate is easily formed. In particular, a molecular weight of less than 20,000 (2.0×10⁴ g/mol) is preferable in terms of exosome collection efficiency.

The molecular weight dispersity (PDI) of the copolymer is not particularly limited, but from the viewpoint of obtaining a copolymer having more excellent effects of the present disclosure, the molecular weight dispersity is preferably 1.5 or less and more preferably 1.3 or less.

The copolymer may include repeating units other than the above. Examples of the repeating units other than the above include repeating units based on N-cyclopropylacrylamide (LCST: 46°C), N-n-propylacrylamide (LCST: 22°C), N-tetrahydrofurfuryl acrylamide (LCST: 28°C), N-ethoxyethylacrylamide (LCST: 35°C), N-methyl-N-ethylacrylamide (LCST: 56°C), N-methyl-N-isopropylacrylamide (LCST: 23°C), N-methyl-N-n-propylacrylamide (LCST: 20°C), N,N-diethylacrylamide (LCST: 32°C), N-cyclopropylmethacrylamide (LCST: 59°C), N-isopropylmethacrylamide (LCST: 44°C), N-n-propylmethacrylamide (LCST: 28°C), and N-tetrahydrofurfurylmethacrylamide (LCST: 35°C).

The copolymer can be synthesized by, for example, carrying out the following procedures 1 and 2 in this order. In the procedure 1, a precursor copolymer is obtained by copolymerizing monomers represented by the following formulae 1' and 5'.

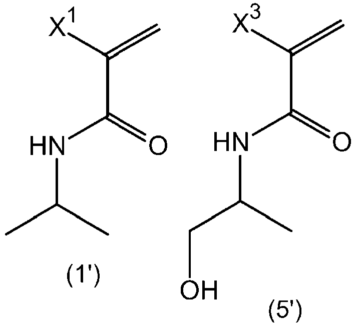

X¹ in the formula 1' and X³ in the formula 5' are synonymous with X¹ in the formula 1 and X³ in the formula 5, respectively, and preferable forms thereof are also the same.

The method for copolymerizing the above monomers is not particularly limited, and living polymerizations, such as living radical polymerization, living anionic polymerization, and living cationic polymerization, can be preferably used. In particular, from the viewpoint of obtaining a precursor copolymer more simply, the living radical polymerization is preferable.

The living radical polymerization is based on establishing a rapid equilibrium of a small amount of a propagating radical (free radical) species and a large amount of a dormant species in the growth reaction by using heat, light, a metal catalyst, and so on. Various forms of living radical polymerization using dormant chains have been proposed.

For example, an atom transfer radical polymerization (ATRP) method using an alkyl halide as a dormant, a reversible addition fragmentation chain transfer (RAFT) method using a thioester, and a nitroxide mediated polymerization (NMP) method using an alkoxyamine are known.

The RAFT method is a method of polymerizing a vinyl monomer by adding a chain transfer agent called a RAFT agent having a high chain-transfer constant to a general radical polymerization system. As the RAFT agent, a thioester can be used.

The amount of the RAFT agent can be appropriately selected by the molecular weight of the target copolymer. That is, in order to bind the RAFT agent to an end of each copolymer, for example, when a 100mer copolymer is a target material, the RAFT agent may be used in an amount of 0.1 to 3 mol% with respect to 100 mol% of a monomer.

The radical polymerization initiator that is used in the RAFT polymerization is not particularly limited, and may be appropriately selected from known initiators such as an azo compound, a peroxide, and a redox type.

Examples of the azo compound include 2,2'-azobisisobutyronitrile (AIBN), 2,2'-azobis-2,4-dimethylvaleronitrile, and 2,2'-azobis(2-methylpropionamidine) dihydrochloride, and 4,4'-azobis(4-cyanovaleric acid).

The amount of the polymerization initiator is generally preferably 0.1 to 50 mol% based on 1 mole of the RAFT agent. The reaction of the RAFT method is determined based on the radical polymerization initiator to be used, but is generally often carried out at 40°C to 150°C. Polymerization is frequently carried out under atmospheric pressure, but is possible even under pressure.

The RAFT method can be carried out in the absence of a solvent, but also can be carried out in the presence of a solvent. The solvent that is used as needed is not particularly limited, and a known solvent can be used. It is also possible to carry out the reaction in water, and the reaction progresses in also emulsion polymerization. As the emulsifier that is used in such a reaction, a nonionic emulsifier, a cationic emulsifier, and an anionic emulsifier that can be used in general emulsion polymerization can be used.

Regarding the synthesis procedure of a precursor copolymer again, a process of obtaining a copolymer is implemented as the procedure 2 after the procedure 1 by reacting the precursor copolymer and a precursor compound represented by a formula 10: RAk-L¹⁰-COOH. In the formula 10, RAk represents the above-mentioned cyclic alkynyl group, and L¹⁰ represents a divalent linking group, and specifically, a group that is the same as L¹ in the formula 2 can be adopted.

A desired copolymer is synthesized by forming an ester bond (condensation) from the hydroxy group possessed by the precursor copolymer and the carboxy group possessed by the precursor compound represented by the formula 10.

The method for forming an ester bond is not particularly limited, and examples thereof include a method of performing a condensation reaction of a precursor copolymer and a precursor compound in the presence of a condensing agent, a catalyst, and a solvent at 0°C to 150°C (preferably 0°C to 100°C) for from 30 minutes to 24 hours (preferably 3 to 15 hours).

As the condensing agent, triphenyl phosphite, N,N'-dicyclohexylcarbodiimide, 1-ethyl-3-(3-dimethylaminopropyl)carbodiimide hydrochloride, N,N'-carbonyldiimidazole, dimethoxy-1,3,5-triazinylmethylmorpholinium, O-(benzotriazol-1-yl)-N,N,N',N'-tetramethyluronium tetrafluoroborate, O-(benzotriazol-1-yl)-N,N,N',N'-tetramethyluronium hexafluorophosphate, and diphenyl (2,3-dihydro-2-thioxo-3-benzoxazolyl)phosphonate can be used. In particular, N,N'-dicyclohexylcarbodiimide is preferable. In this case, as the catalyst, N,N-dimethyl-4-aminopyridine can be used, and as the solvent, dichloromethane or the like can be used.

Regarding the description of Step S10 in Fig. 1 again, a ligand molecule will now be described. Examples of the ligand molecule include a protein, an antibody, a peptide, and derivatives thereof each having a binding group that can bind to the reactive group of a linker described later. The ligand molecule is preferably a molecule that can specifically bind to a molecule (marker) present in the outer membrane of an exosome.

In the present specification, the "protein" means a polymer compound composed of amino acids linked by peptide bonds. Examples of the protein include biological molecules having various functions, such as an enzyme, a hormone, and a structural protein. The protein generally forms a specific three-dimensional structure depending on the function. In the present specification, the "antibody" is defined as a group different from the above-described "protein". The antibody is known as an immunoglobulin (Ig) and means a protein having an ability of binding to a specific antigen. In the present specification, a molecule that binds to a target by an antigen-antibody reaction is defined as "antibody", and other proteins are defined as "protein".

As the ligand molecule that is an antibody, an antibody against a marker present in the outer membrane of an exosome can be used. Examples of the antibody include anti-CD63 antibody, anti-CD81 antibody, and anti-CD9 antibody targeting CD63, CD81, and CD9 known as tetraspanin markers present in exosome membrane. These antibodies include an amino group (e.g., lysine residue) that can bind to an active ester group or the like and can be easily bonded to a linker to form a conjugate as a result.

An antibody directed against a target specific to derived cells can be used in some cases. For example, an exosome derived from epithelium cells may be collected using an EpCAM antibody. Exosomes derived from HER2-positive cancer may be collected using an HER2 antibody. In addition, exosomes derived from EGFR-related cancer may be collected using an EGFR antibody. For cancer cell-derived exosomes, a PD-L1 antibody can be used in some cases. In addition to the above, antibodies targeting immune-related molecules and transport-related molecules can be used as ligand molecules.

As the ligand molecule that is a peptide, a peptide called "Vn96" or the like that targets heat shock proteins (HSP70 and HSP90) present in the exosome outer membrane can be used.

As the ligand molecule that is a protein, proteins targeting respective targets exposing to the outer membrane of an exosome can be used. For example, proteins targeting a phosphatidylserine group and heat shock proteins (HSP70 and HSP90) can be used. Examples of the protein targeting the phosphatidylserine group include Annexin family (e.g., Annexin I, II, IV, V, and VI), prothrombin, β2-GPI, Tim-4 and MFG-E8. Protein-based ligand molecules often have lower affinity for their targets compared to the antigen-antibody reaction. Examples of the binding form include electrostatic interaction, hydrophobic interaction, hydrogen bonding, and metal ion-dependent binding, and the use of these ligand molecules that are proteins dissociate the exosome immobilized to a conjugate and preferably easily collect only the exosome. In particular, the use of a metal ion-dependent binding ligand molecule is preferable because an exosome can be more easily collected by adjusting the metal ion concentration by a chelating agent.

For example, Annexin V, prothrombin, or Tim-4 binds to a phosphatidylserine group as a marker, metal ion (calcium ion) independently. Accordingly, an exosome binding with a conjugate can be easily separated from the conjugate by decreasing the calcium ion concentration by a chelating agent (such as EDTA).

Regarding Step S10 in Fig. 1 again, the linker will now be described. The linker is a compound including an azido group that can react with RAk of the copolymer by [3+2] cycloaddition reaction and further including a reactive group that can bind with the ligand molecule. Specifically, the linker is preferably a compound represented by a formula 4:

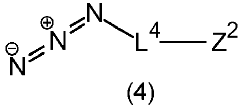

In the formula 4, L⁴ is a divalent linking group and is the same as L¹, and preferable forms thereof are also the same. In the formula 4, Z² represents a reactive group that can bind with a ligand molecule.

When the ligand molecule includes an amino group, as Z², an NHS ester group (N-hydroxysuccinimide ester group), an isothiocyanate group, and so on can be adopted. These groups can form a stable bond by a reaction with an amino group. In addition, Z² may be a carboxyl group. The formation of an amide bond with an amino group of the ligand molecule can be accelerated by using a carbodiimide compound (EDC or DCC) in combination.

When the ligand molecule includes a mercapto group, Z² may be a maleimide group. The maleimide group binds selectively to a mercapto group of a cysteine residue and can form a stable thioether bond. As Z² that can bind to a mercapto group, a vinylsulfonic acid group, a halogenated alkyl group, and so on can also be used.

When the ligand molecule includes a histidine residue, a tyrosine residue, and a tryptophane residue, Z² may be a glycidyl group. From the viewpoint of more easily obtaining a conjugate, the group represented by Z² of the linker is preferably a reactive group that can react with an amino group, a mercapto group, or a carboxyl group as a binding group of the ligand molecule. Specifically, an active ester group (such as an NHS ester group), an isothiocyanate group, a carboxyl group, a maleimide group, and so on are preferable.

Examples of the preferable linker include a compound represented by the following formula 41. In the formula 41, L⁴ represents a divalent linking group and is the same as L⁴ in the formula 4.

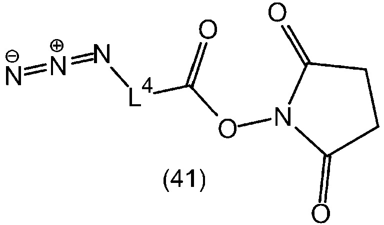

The linker binds to a ligand molecule through the reactive group represented by Z² and, at the same time, binds to a cyclic alkynyl group possessed by a copolymer through an azido group. Thus, a copolymer and a ligand molecule can be bound to each other through a triazole ring.

The exosome-collecting method of Fig. 1 will be described again. After the completion of preparation of a copolymer, a linker, and a ligand molecule in Step S10, a linker-ligand molecule complex is then formed in Step S11. In a method for forming the linker-ligand molecule complex, for example, a linker represented by the formula 4 is dispersed in water, an organic solvent, or a mixture solution thereof, and a ligand molecule may be further added thereto. The ligand molecule may be dispersed in a carbonate buffer solution or the like, and the dispersion may be added to a solution obtained by dissolving a linker in an aprotic organic solvent such as dimethylsulfoxide.

The reaction temperature is not particularly limited, but is preferably 1°C to 20°C and more preferably 1°C to 10°C. The reaction time is not particularly limited, but is preferably 1 to 24 hours. The addition ratio of the ligand molecule and the linker when the linker-ligand molecule complex is formed is not particularly limited, but the substance amount-based ratio of the reactive group (Z²) to the content of the corresponding binding group possessed by the ligand molecule, i.e., an amino group, a mercapto group, a carboxyl group, and so on, (molar ratio, reactive group/binding group), is preferably 0.1 to 500. The addition amount of the linker to 1 mole of the ligand molecule is not particularly limited but is preferably 0.01 to 5,000 mole, more preferably 1 to 2,000 mole, and further preferably 50 to 200 mole.

In Step S12, a conjugate of the copolymer and a linker-ligand complex is formed. The conjugate to be formed is preferably a compound including repeating units represented by the following formula 1 and the following formula 6.

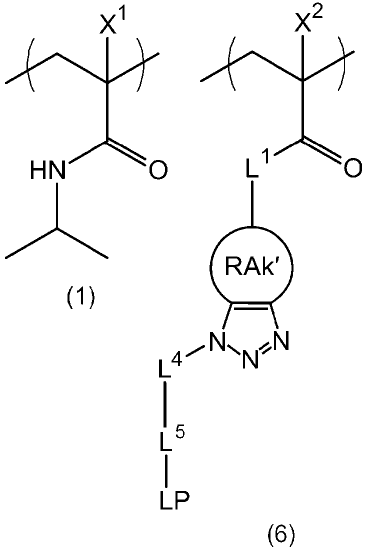

The unit 1 represented by the formula 1 is a unit constituting the copolymer as already described, and preferable forms thereof are also the same. In the formula 6, X² is synonymous with X² in the formula 2, and preferable forms thereof are also the same. In addition, L⁴ and L⁵ represent divalent linking groups that may be the same or different. Among them, L⁴ has the same meaning as that of the same symbol in the formula 4. That is, L⁴ is a divalent linking group that is derived from the linker and introduced into the unit 6. LP is a monovalent group derived from the ligand molecule. In other words, LP is a ligand molecule residue.

The divalent linking group as L⁵ is a divalent group that is generated by binding of the binding group of a linker and the reactive group of a ligand molecule. Specifically, examples of the divalent linking group include groups represented by -C(O)NH-, -S-, -C(O)O-, -NH-C(O)-NH-, -O-, -S-S-, and -S-CₙH₂ₙ- (n is an integer of 1 or more). For example, when the reactive group of the linker is a carboxyl group and the binding group of the ligand molecule is an amino group, L⁵ is -C(O)NH-. When the reactive group of the linker is a maleimide group and the binding group of the ligand molecule is a mercapto group, L⁵ is -S-. When the reactive group of the linker is a carboxyl group (or its ester) and the binding group of the ligand molecule is a hydroxy group, L⁵ is -C(O)O-. When the reactive group of the linker is an isothiocyanate group (-N=C=S) and the binding group of the ligand molecule is an amino group, L⁵ is -NH-C(O)-NH-. When the reactive group of the linker is an epoxy group and the binding group of the ligand molecule is a hydroxy group, L⁵ is -O-.

In the formula 6, the group represented by RAk' is a residue of a cyclic alkynyl group RAk bound to the 4-position of a stable 1,4-substituted triazole that is formed by binding the cyclic alkynyl group RAk included in the unit 2 represented by the formula 2 and the azide of the linker through [3+2] cycloaddition reaction. Since the structure other than the alkynyl group that is incorporated into the triazole ring does not change in the [3+2] cycloaddition reaction, the structure of RAk' is the same as RAk in the portion other than the alkynyl group, and preferable forms thereof are also the same.

The conjugate preferably includes a unit 1 represented by the formula 1 and a unit represented by the following formula 61. In the formula 61 and the formula 6, X² is synonymous with X² in the formula 2, and preferable forms thereof are also the same. L⁴ and L⁵¹ represent divalent linking groups that may be the same and different. Among them, L⁴ has the same meaning as that of the same symbol in the formula 4. That is, L⁴ is a divalent linking group that is derived from the linker and introduced into the unit 6. L⁵¹ is preferably -NH- or -O-. LP is a monovalent group derived from the ligand molecule. In other words, LP is a ligand molecule residue. RAk' has the same meaning as that of the same symbol in the formula 6, and preferable examples thereof are also the same.

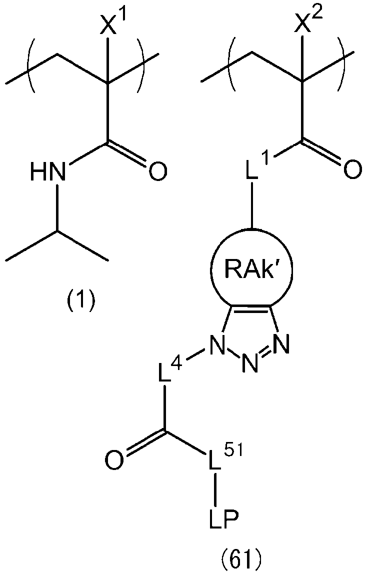

Examples of the structure that is formed by binding the cyclic alkyl group RAk included in a unit 2 represented by the formula 2 and the azide of the linker through [3+2] cycloaddition reaction include a structure represented by the following formula 9 and a structure obtained by introducing a monovalent or divalent substituent that can be added to RAk into a structure represented by the formula 9. In the formula 9, * binding to the nitrogen atom at the 1-position of triazole represents the binding position with the divalent linking group represented by L⁴, and the other * represents the binding position with the divalent linking group represented by L¹.

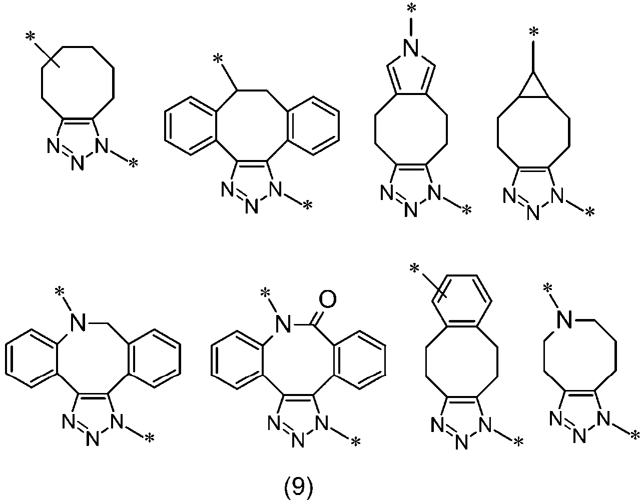

In the conjugate, the mole-based content ratio of the content of the copolymer to the content of (the group LP derived from) the ligand molecule is not particularly limited, but is preferably 0.1 to 100.0, 0.5 to 70.0, 0.5 to 50.0, 0.5 to 30.0, 1.0 to 50.0, 1.0 to 40.0, 2.0 to 100.0, 2.0 to 50.0, 5.0 to 100.0, 5.0 to 50.0, 10.0 to 100.0, 10.0 to 50.0, or 10.0 to 30.0.

The method for forming the conjugate is not particularly limited, but the conjugate may be formed by mixing and stirring a linker-ligand molecule complex and a copolymer in water containing a buffering agent as needed. The reaction temperature is not particularly limited, but is preferably 1°C to 20°C and more preferably 4°C to 10°C. The reaction time is not particularly limited, but is preferably 1 to 24 hours.

In Fig. 1, Step S11 and Step S12 are different procedures, but Step S11 and Step S12 may be performed simultaneously, or a procedure flow of binding a copolymer and a ligand molecule and then immobilizing the ligand molecule can also be adopted. In all of the cases, it is sufficient that a conjugate can be formed by conjugating three components prepared in Step S10.

Then, in Step S13, the conjugate is brought into contact with an exosome in a mixture solution thereof. In this step, the group (LP) derived from the ligand molecule of the conjugate binds to (the target of the outer membrane of) an exosome, and the exosome is immobilized to the conjugate.

The mixture solution can be prepared by adding the conjugate to a sample, such as a human body fluid and a cell culture medium, containing an exosome. This is an example, and it is sufficient that the mixture solution contains at least the conjugate and the exosome. This procedure flow corresponds to the form in which a conjugate is prepared in advance (until Step S12) and a sample is added thereto. Alternatively, a sample may be added to a solution of a mixture of a copolymer, a linker, and a ligand molecule, or a mixture solution may be prepared by mixing a copolymer, a linker, a ligand molecule, and a sample simultaneously.

On this occasion, a copolymer in a free state (not forming a conjugate) may be further added to the mixture solution. The addition of a copolymer makes the response to heat more sensitive and can concentrate an exosome more efficiently. The addition amount of the copolymer is not particularly limited, but when the mole-based content of the copolymer contained in the conjugate is defined as 1, the content of the copolymer is preferably 0.1 to 10.

Then, in Step S14, the mixture solution is heated to aggregate the conjugate capturing an exosome to separate the conjugate from the mixture solution. The mixture solution is heated to a temperature not lower than the aggregation temperature set in advance based on the lower critical solution temperature (LCST) of the copolymer to aggregate the copolymer. There is an additive correlation between the LCST of each unit included in a copolymer and the content (mol%) of each unit, and the LCST of a copolymer can be estimated depending on the composition of the copolymer. Specifically, 20°C to 40°C is preferable. The method for solid-liquid separation is not particularly limited, but centrifugation may be performed under usual conditions. As an example, a rotation speed of 1,000 to 15,000 rpm, a centrifugal acceleration of 100 to 50,000 ×g, and a period of time of 1 to 40 minutes may be adopted. The temperature on that occasion is not particularly limited, but may be 20°C to 40°C.

Then, in Step S15, a chelating agent is acted on the solid obtained in Step S14, the conjugate and the exosome are separated, and the conjugate is then removed to obtain the exosome.

When the ligand molecule is a metal ion-dependent binding protein, the captured exosome can be dissociated by a chelating agent. The chelating agent can be appropriately selected depending on the type of the metal ion that contributes to the binding. When the metal ion that contributes to the binding is a calcium ion, ethylenediaminetetraacetic acid (EDTA), ethylene glycol bis(β-aminoethyl ether) tetraacetic acid (EGTA), 1,2-bis(o-aminophenoxy)ethane-N,N,N',N'-tetraacetic acid (BAPTA), and so on can be used.

The addition amount of the chelating agent is not particularly limited, but it is preferable to consider the metal ion concentration and adjust the addition amount to a molar concentration exceeding the metal ion concentration. A buffering solution (or buffer solution) may be added together with the chelating agent so as to obtain a pH level according to the chelating agent. Specifically, when EDTA is used as the chelating agent, the pH level may be adjusted by a buffering solution such that the pH is about 7.5. Examples of the buffering solution include Tris-HCl (tris(hydroxymethyl)aminomethane hydrochloride), HEPES (4-(2-hydroxyethyl)-1-piperazine ethanesulfonic acid), PBS (phosphate buffered saline), and MOPS (3-(N-morpholino)propanesulfonic acid).

The method for acting a chelating agent on the conjugate capturing the exosome is not particularly limited, and examples thereof include a method of adding a buffer solution containing the chelating agent to the above-described solid and mixing and stirring it. The reaction temperature on this occasion is not particularly limited, but is preferably 1°C to 20°C and more preferably 4°C to 10°C. The reaction time is not particularly limited, but is preferably 1 to 24 hours.

Since the conjugate and the exosome are separated from each other by the above reaction, subsequently, centrifugation is performed under usual conditions (which may be the same as in Step S14), and the aggregated conjugate is removed to collect a solution containing the captured exosome. The method in this example can be adopted when the ligand molecule is protein-based and forms metal ion dependent binding. When the ligand molecule is protein-based and the bond dissociates in a temperature dependent matter, instead of this procedure, a procedure for adjusting the temperature may be further performed. According to this method, the exosome can be collected more easily in a complete form compared to when the ligand molecule is antibody-based.

In contrast, when the ligand molecule is antibody-based, this step (Step S15) can be omitted. In antibody-based, it is more suitable for analysis that does not require consideration of shape and structure while collecting a highly pure exosome.

### [Exosome collection kit]

The exosome collection kit of the present disclosure at least includes the above-described copolymer, linker, and ligand molecule and may be a configuration further including an appropriate solvent and other necessary reagent and material. The exosome collection kit is designed for the purpose of efficiently collecting a specific exosome, and a set may be packaged considering the convenience in a research or clinical setting.

Specifically, the exosome collection kit can be used for treating a predetermined amount of a sample containing exosomes and collecting an exosome as a target highly efficiently. The exosome collection kit includes a copolymer, a linker, a ligand molecule prepared at an appropriate ratio in advance and also includes a stabilizer and a storage solvent for preventing decomposition or inactivation of each component. The exosome collection kit further includes a protocol (operation procedure manual) for making the operation easy in the same package, and is designed to be easily operated by researchers and medical professionals using standard equipment.

The components in the exosome collection kit are accommodated in respective appropriate containers and are designed so that a necessary amount can be clearly measured. The exosome collection kit may include a buffer solution, a cleaning solvent, a chelating agent, an auxiliary reagent, and so on that are necessary when exosomes are collected, which minimizes the need for additional preparation by the user.

Furthermore, the exosome collection kit is designed so as to be suitable for specific research or clinical testing application and is optimized for enhancing the capturing efficiency of an exosome as a target. For example, when metal ion dependent binding is used, it is also possible to adopt a configuration including an appropriate metal ion solution and a chelating agent for its separation. Consequently, the user can separate and collect an exosome contained in a sample specifically and efficiently and can quickly proceed to subsequent analysis or application.

Such an exosome collection kit is expected to be widely used in basic research, diagnostic research, and therapeutic development as an important tool in the field of exosome research. In particular, the kit can also contribute to the advancement of next-generation medical diagnostic technology and personalized medicine because a target exosome can be captured and separated simply and with high reproducibility.

### [Conjugate preparation kit]

The conjugate preparation kit of the present disclosure is a kit for preparing a conjugate (combination) specific for exosome capturing using a ligand molecule selected by the user. The conjugate preparation kit does not include a ligand molecule and has a configuration including a copolymer, a linker, a necessary solvent, a buffer solution, and an auxiliary reagent depending on the binding group of a ligand molecule.

The conjugate preparation kit is characterized in that variations are available depending on the binding group of a ligand molecule. For example, when the ligand molecule has the following binding group, specifications respectively corresponding to each are provided. That is, when the binding group is an amino group (-NH₂), the conjugate preparation kit is a "conjugate preparation kit (for amino group)" including a linker including an NHS ester group or the like. When the binding group is a carboxyl group (-COOH), the conjugate preparation kit is a "conjugate preparation kit (for carboxyl group)" that includes a linker including a hydroxy group or the like and may include a dehydrating condensing agent such as an EDC, e.g., a carbodiimide compound, as an auxiliary reagent. When the binding group is a mercapto group (-SH), the conjugate preparation kit is a "conjugate preparation kit (for mercapto group)" that may include a linker including a maleimide group. When the binding group is an aromatic group such as a phenol group, the conjugate preparation kit is a "conjugate preparation kit (for aromatic group)" that may include a linker including an epoxy group or the like. By using the conjugate preparation kit, the user can select an optimum ligand molecule according to the purpose of research and experiment and prepare a conjugate efficiently and flexibly.

Furthermore, the conjugate preparation kit includes a detail protocol in the same package, and the user can produce a conjugate with high reproducibility by following the operation procedure depending on the type of the ligand molecule. Since the copolymer and the linker can be supplied in a form dissolved in an appropriate solvent considering the storage and stability properties, the effort for use is minimized. Such a conjugate preparation kit can be widely utilized in the fields of research and medicine as a tool for efficiently capturing a specific exosome.

### [Conjugate and application of collected exosome]

An exosome can be collected while keeping the shape and structure of the exosome by using the conjugate of the present disclosure. Conventional methods have problems of deformation of an exosome and disruption of the membrane structure due to an excessive centrifugal force and chemical treatment. However, highly efficient collection by minimizing these effects is possible by using the conjugate of the present disclosure. Consequently, the internal components of an exosome and molecules on the membrane can be correctly analyzed, and the following applications are realized.

The first is application to pathological diagnosis. Since the exosome collected using the conjugate of the present disclosure keeps its shape and membrane structure, it is possible to detect a specific disease-related biomarker with high sensitivity. Consequently, the conjugate contributes to early diagnosis and progression assessment of cancer, nervous disease, infectious disease, and so on. Elucidation of the pathological mechanism of disease and contribution to personalized medicine are also expected through analysis of membrane molecules and internal RNA and protein of an exosome.

The second is application to analysis of a disease biomarker. By using the conjugate of the present disclosure, an exosome can be specifically captured and separated, and a small amount of a disease biomarker in a sample can be efficiently concentrated. For example, it is possible to detect disease and monitor the progression by collecting an exosome derived from a tumor and analyzing a molecule that is expressed on the membrane or a specific RNA included inside the membrane. This method exhibits high specificity and sensitivity compared to conventional detection methods and is an important tool in research and clinical applications.

The third is application to a drug delivery system (DDS). The exosome specifically collected through the conjugate of the present disclosure can also be used as a drug delivery vector. The exosome is gaining attention as efficient delivery means for a therapeutic agent because of its high biocompatibility and natural immune evasion property. Drug delivery to tumor cells or specific target tissue can be realized by using the conjugate of the present disclosure, and applications to cancer treatment or intractable disease treatment are expected.

The fourth is application to a method for detecting an RNA sequence. The RNA included inside an exosome plays an important role as a disease-related biomarker. Detection of disease, selection of treatment method, and also prognostic monitoring are possible by extracting RNA from the exosome collected using the conjugate of the present disclosure and analyzing the sequence of the RNA. Damage of the RNA can be prevented by keeping the shape and structure of the exosome, which allows for more accurate sequence detection.

The fifth is application to research and technology development. Characteristic analysis and molecular dynamics research of exosomes are promoted by using the conjugate of the present disclosure. Consequently, it is possible to find a new role of an exosome in basic research or to develop next-generation diagnostic and therapeutic technologies using exosomes.

### [Automated device]

The automated device of the present disclosure is an automated device using the exosome collection kit. The automated device is designed for the purpose of collecting exosomes contained in a sample efficiently and with high reproducibility. In conventional methods, the operation procedure is complicated, which can result in a decrease in the collection efficiency. However, stable collection can be realized while keeping the shape and function of the exosome by using the automated device.

The automated device includes a reagent supply unit, a reaction control unit, a separation and collection unit, and an output unit that are controlled by a controller such as a programable controller.

The reagent supply unit is a unit for supplying the reagents included in the exosome collection kit in an appropriate order. This unit includes a holder for retaining a plurality of reagent containers, a dispenser for supplying an appropriate amount of a reagent, and a control module for controlling the supply process. The supply of a reagent is carried out automatically depending on the progress of the collection process.

The reaction control unit is a unit for optimizing the conditions for the reaction of an exosome and a conjugate. This unit has functions for controlling the temperature, agitation speed, and reaction time and plays a role for enhancing the efficiency of the reaction. The reaction container to be used is designed to enable uniform mixing.

The separation and collection unit is a unit for capturing an exosome and removing impurities. This unit includes a solid-liquid separation mechanism such as a centrifugation mechanism and can remove unnecessary components in a sample and then separate the collected exosome with a high purity. Highly reproducible results can be obtained, while preventing the damage of the collected exosome, by automating the cleaning process.

The output unit is a unit for accommodating the collected exosome in an appropriate container and outputting it in a storable condition. This unit has a function of controlling the temperature and storage environment such that the collected exosome does not deteriorate.

The method for using the automated device is as follows. A sample is set in a reaction container in the device. Subsequently, the reagent supply unit is operated to supply reagents in an appropriate order. Subsequently, the reaction control unit is operated to carry out the binding reaction of an exosome and a conjugate at an optimum condition. Subsequently, the separation and collection unit is operated to collect an exosome and also remove unnecessary components. Subsequently, the output unit is operated to accommodate the collected exosome in a storage container.

The use of an automated device can efficiently collect an exosome from a sample and obtain the exosome with a high purity without deteriorating the shape and function. Consequently, utilization of an exosome in research and diagnostic applications can be further promoted.

### [Analysis system]

The analysis system of the present disclosure is an integrated system that can implement the exosome-collecting method of the present disclosure. The analysis system is purposed for implementing the collection and analysis of an exosome as a series of processes and is composed of a plurality of modules for efficiently performing capturing and separation of an exosome from a sample and subsequent detail molecular analysis. Specifically, the analysis system includes an exosome capture module, a molecular analysis module, a data processing module, and an interface module. Each module may be controlled by a controller.

The exosome capture module is a module for efficiently capturing an exosome contained in a sample using the exosome collection kit of the present disclosure. The above-described automated device can use the module as an exosome capture module. The exosome capture module captures specifically a specific exosome from a sample.

The molecular analysis module is a module for analyzing the molecule (examples: protein, RNA, and so on) included in the captured exosome. This module has the following functions:
(1) Immunological method for quantitative and qualitative analyses of an exosome surface molecule (example: ELISA);
(2) Method of extracting exosome internal RNA and carrying out sequence analysis by next-generation sequencing (NGS) or real-time PCR; and
(3) Precise protein analysis method using a mass spectrometer.

The data processing module has functions of processing the data obtained by the molecular analysis module and of generating analysis results according to the purpose. This module may include software statistically processing analysis data.

The interface module carries out setting the sample amount and analysis conditions, monitoring the experiment process, and outputting the result. The interface module may further have a data sharing function through remote control or network.

The method of using the analysis system is as follows. A sample is set in the exosome capture module, and appropriate conditions are set. Subsequently, the exosome capture module automatically collects an exosome from a sample. Subsequently, the captured exosome is transferred to the molecular analysis module, and the internal components and surface molecules are analyzed. Subsequently, the analysis results are processed by the data processing module, and the ultimate diagnosis information or research results are output through the interface module.

The system provides integrated processes from the collection of an exosome to the molecular analysis and realizes simplification of the manual work and high reliability of data. Consequently, in application to basic research, diagnostic research, or medical setting, a new technological innovation using an exosome can be promoted.

### Examples

Copolymers and so on of the present disclosure will be described based on the experimental results. The technical idea of the present disclosure should not be interpreted to be limited to the following specific experimental examples.

### (Experimental procedure)

### 1. Synthesis of HIPAAm monomer

2-Hydroxy-N-isopropylacrylamide (HIPAAm) was synthesized by the following procedure. 2-Amino-1-propanol and triethylamine were dissolved in chloroform in an equimolar ratio and were stirred at -5°C. Subsequently, acryloyl chloride was carefully added to the reaction mixture at the same molar ratio, and stirring was continued at -5°C for 2 hours or more. Chloroform was substituted with 2-propanol, and the solution was stored in a freezer for 1 day or more. The salt was removed by filtration, and the reaction product was then concentrated and purified by column chromatography. The final product was analyzed by thin-layer chromatography (TLC) and ¹H NMR to verify the synthesis of HIPAAm.

### 2. Synthesis of temperature-responsive polymer Poly(NIPAAm-co-HIPAAm)

A temperature-responsive polymer Poly(NIPAAm-co-HIPAAm) was synthesized by reversible addition-fragmentation chain transfer polymerization (RAFT polymerization) using temperature-responsive N-isopropylacrylamide (NIPAAm) and a previously synthesized HIPAAm monomer. NIPAAm (1.57 g), HIPAAm (0.44 g), cyanomethyl dodecyl trithiocarbonate (CDT, 12.7 mg), 2,2'-azobis(isobutyronitrile) (AIBN, 1.31 mg), and ethanol (17.6 mL) were mixed and stirred at 60°C for 24 hours. Subsequently, purification was performed. The reaction scheme is as follows.

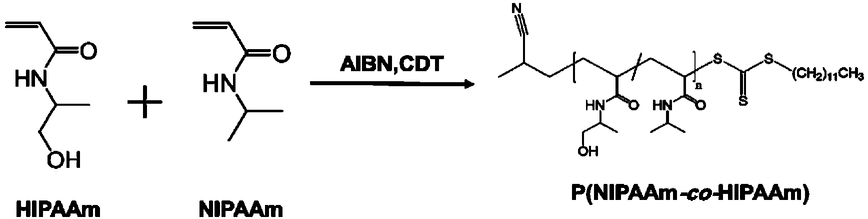

### 3. Synthesis of temperature-responsive polymer Poly(NIPAAm-co-HIPAAm-co-SAKIPAAm)

The alkyne group (cyclic alkynyl group) of dibenzocyclooctyne acid (DBCO acid) was introduced into a temperature-responsive polymer Poly(NIPAAm-co-HIPAAm), and a temperature-responsive polymer Poly(NIPAAm-co-HIPAAm-co-SAKIPAAm) capable of click reaction was synthesized using a dehydration condensation reaction. Poly(NIPAAm-co-HIPAAm) (100 mg), DBCO acid (35.8 mg), 4-dimethylaminopyridine (DMAP, 12 mg), dicyclohexylcarbodiimide (DCC, 20 mg), and dichloromethane (DCM, 30 mL) were mixed and stirred at room temperature overnight and were then purified. The reaction scheme was as follows. A DBCO-introduced unit is defined as SAKIPAAm.

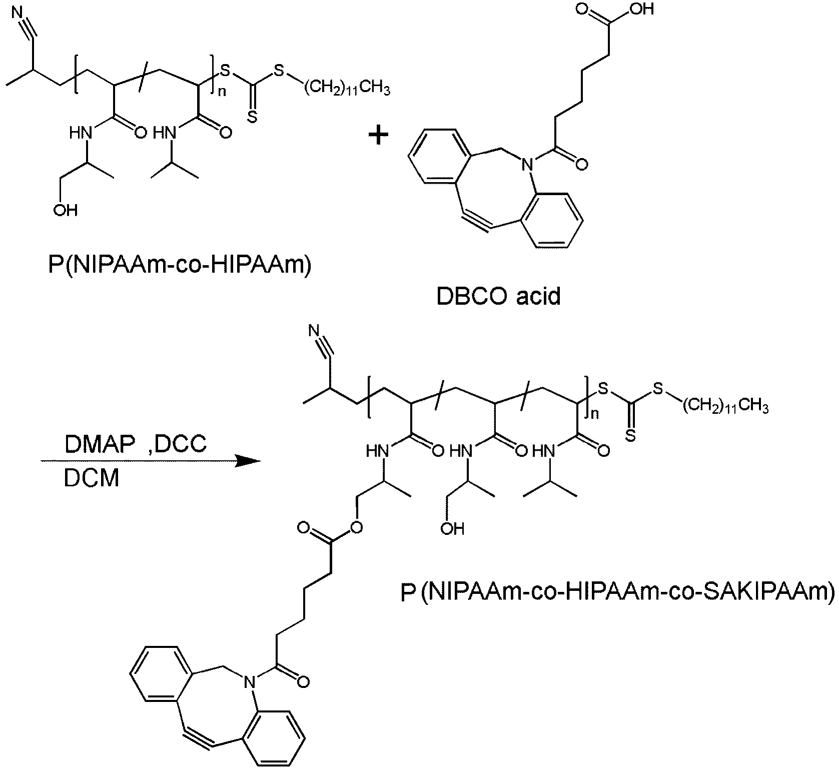

### 4. Characteristic evaluation of polymer synthesis

Whether a temperature-responsive polymer Poly(NIPAAm-co-HIPAAm) and its clickable form Poly(NIPAAm-co-HIPAAm-co-SAKIPAAm) were synthesized successfully or not was verified by ¹H NMR analysis (D₂O and DMSO were used as solvents). A change in the molecular weight was evaluated by gel permeation chromatography (GPC) (DMF and polystyrene were used as a solvent and a standard, respectively). The temperature responsivity of each polymer was evaluated by measuring the lower critical solution temperature (LCST) at a polymer concentration of 2.0 mg/mL, using a solvent PBS (pH 7.4) within a temperature range of 10°C to 40°C.

### 5. Binding azido group and anti-CD81, anti-CD9, and Tim-4 protein

An azido-PEG4-NHS ester was dissolved in DMSO (10 mg/mL), and a dilution series of the azido-PEG4-NHS ester was produced using a carbonate buffer solution of pH 8.6 prepared in advance. Azide was added to an antibody or protein at different supply ratios, and the mixtures were reacted at 4°C for 5 hours while rotating.

### 6. Verification of binding of azido group with anti-CD81, anti-CD9, and Tim-4 protein

After the above-described reaction, an azide-labeled antibody or protein sample (concentration: 0.5 mg/mL, 50 µL) was added to a microplate, and a dilution series of D,L-2-aminobutyric acid was arranged as a standard in parallel. Fluorescamine (50 mg/mL, 5 µL) was added to each well and was incubated for 15 minutes, and the fluorescence intensity at 395 nm or 495 nm was then measured for verifying the binding.

### 7. Animal experiment

A liver mesenchymal stem cell (LMSC) and its conditioned medium were separated using 7 to 9-week-old C57BL/6J female mice (35 mice). This research was implemented at National Institute for Materials Science (NIMS), Tsukuba City and carried out according to the guideline in NIMS, Committee for Ethics of Animal Experimentation (Approval No. 62-2020-6).

### 8. Separation of LMSCs and preparation of conditioned medium

LMSCs were separated by a collagenase digestion method, subcultured three times, and then cultured in a complete medium (DMEM containing 10% FBS, 1% antibiotic mixture solution, 1% non-essential amino acids, and 1% L-glutamine) for 1 day. The LMSCs were washed with PBS three times and were then stored in a serum-free basal medium. Then, the supernatant was subjected to centrifugation (from 300 ×g to 10,000 ×g) for removing cell debris, and the obtained conditioned medium (LMSC-CM) was stored at -80°C.

### 9. Collection of LMSCs exosome

An exosome was collected from the conditioned medium (LMSC-CM) by ultracentrifugation (at 100,000 ×g for 90 minutes). The following procedure was applied to a sample as the test object.

### 9-1. Case of using anti-CD81 and anti-CD9:

The azide-modified antibody (1 mg/mL in PBS, 100 µL) was added to a sample (5 mL) and incubated at 37°C for 1 hour. Subsequently, Poly(NIPAAm-co-HIPAAm-co-SAKIPAAm) (antibody: polymer ratio: 1 : 30) was subjected to a click reaction at 4°C for 6 hours. The reaction mixture was centrifugated at 37°C at 13,800 ×g for 5 minutes, and the concentrated precipitate was used for evaluation.

### 9-2. Case of using Tim-4 protein:

An azide-modified protein (1 mg/mL in PBS, 100 µL) and a calcium chloride solution were added to a sample (5 mL) and were incubated at 37°C for 1 hour. Subsequently, Poly(NIPAAm-co-HIPAAm-co-SAKIPAAm) (protein : copolymer ratio: 1 : 30) was subjected to a click reaction at 4°C for 6 hours. The reaction mixture was centrifugated at 37°C for 13,800 ×g for 5 minutes, and Tris-HCl and EDTA buffer were added to the concentrated precipitate. The mixture was stirred at 4°C for 1 hour and then centrifugated at 37°C at 13,800 ×g for 5 minutes, and the released solution was sed for evaluation.

### 10. TEM observation

Transmission electron microscope (TEM) observation was carried out by a negative staining method. A 2% phosphotungstic acid (PTA) aqueous solution (pH 7, adjusted with NaOH) was used as a stain solution, and the TEM grid used was a carbon support film (Cu mesh) and a TEM grid coated with formvar membrane. In hydrophobic treatment of the grid, JIC-410 (Ion Cleaner, manufactured by JEOL Ltd.) was used. A sample was diluted 5-fold with PBS, and 2 µL of the diluted sample was then dropwise applied onto a support film and was left to stand for 1 minute. The excessive sample solution was removed with filter paper, and a PTA solution was then dropwise applied onto the support film and was further left to stand for 1 minute. After staining, the excessive sample solution was removed again with filter paper, and the TEM grid was dried in the air. Subsequently, the TEM grid was stored in a desiccator. Observation with JEM-2100 (manufactured by JEOL Inc.) transmission electron microscope and photographing with an acceleration voltage of 200 kV were performed. In image acquisition, Orius SC200D (CCD camera manufactured by Gatan, Inc.) was used.

### 11. DLS measurement

The exosome solution was diluted with PBS, and the diameter and aggregation state of the sample were measured and evaluated using "Zetasizer-Nano ZSP" (Malvern Panalytical) at 633 nm, a scattering angle of 173°, and a temperature of 25°C.

### 12. SDS-PAGE measurement

After dissolving the elution sample, the protein content was evaluated by comparing with cell lysate by polyacrylamide gel electrophoresis (SDS-PAGE).

### 13. Quantitative measurement of exosome

The total amount of exosomes was quantitatively measured using "ExoQuant" and FluoroCet kit according to the instruction of the manufacturer.

### 14. Statistical analysis

The data were analyzed using SPSS (ver. 21). The descriptive statistics were shown as mean ± standard deviation and compared using ANOVA and paired t-test. The significance level was set at 5% (P < 0.05).

### (Results)

The composition of the synthesized Poly(NIPAAm-co-HIPAAm-co-SAKIPAAm) was as that of Example 6 in Table 1. Other copolymers (Examples 1 to 5 and 7) were synthesized based on International Publication No. WO 2022-219967. The experimental results below were obtained using the copolymer of Example 6 unless otherwise specified. Each copolymer was synthesized by changing the charge ratios of DBCO-acid and so on, and the composition was verified by ¹H-NMR analysis.

| Table 1 | NIPAAm | HIPAAm | SAKIPAAm |
|---|---|---|---|
| Example 1 | 96.4 | 1.2 | 2.4 |
| Example 2 | 96.3 | 1.2 | 2.5 |
| Example 3 | 73.7 | 22.5 | 3.8 |
| Example 4 | 75.5 | 20.4 | 4.1 |
| Example 5 | 87 | 7 | 6.0 |
| Example 6 | 88 | 2.5 | 9.5 |
| Example 7 | 77 | 9 | 14 |

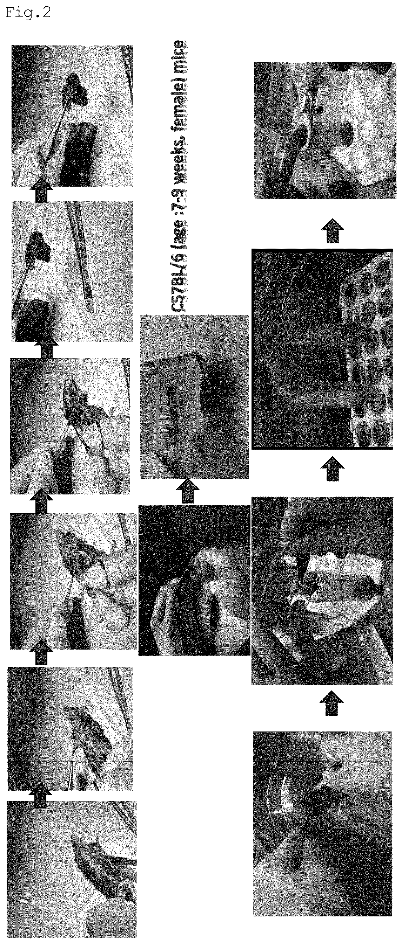

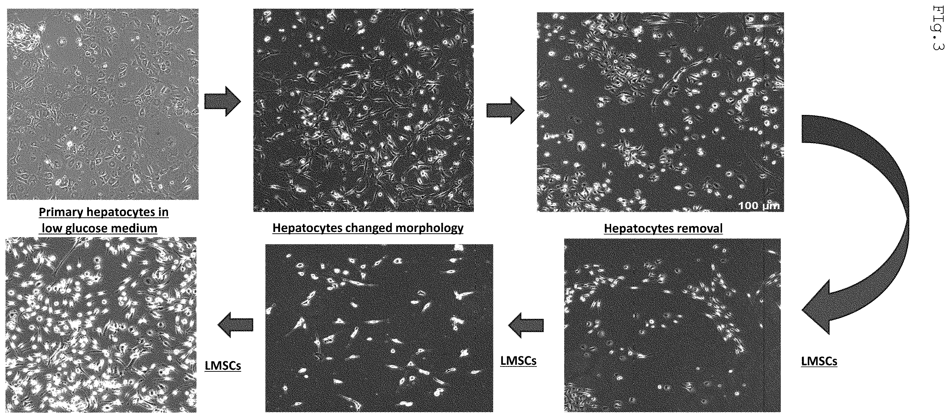

Fig. 2 is an image sequence showing a manner of separating cells from mouse liver in the procedure of extracting mesenchymal stem cells (MSCs). Fig. 3 is an image sequence showing a manner of separating MSCs only by removing stem cells in the procedure of extracting mesenchymal stem cells (MSCs).

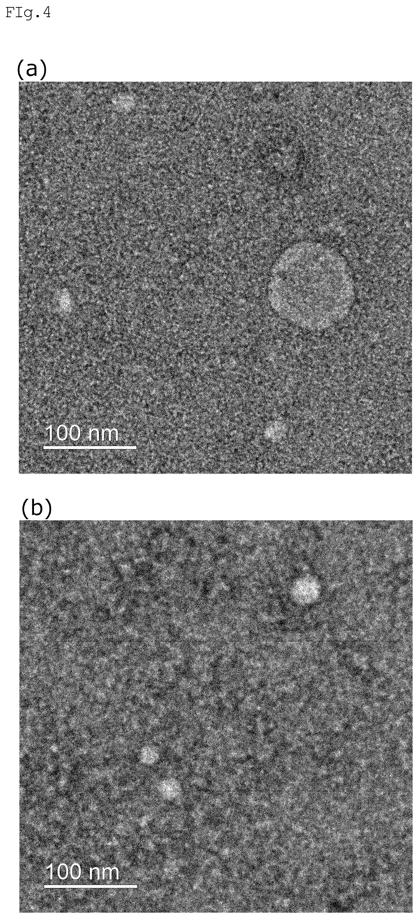

Fig. 4 is TEM images of exosomes collected by ultracentrifugation. Images of (a) and (b) in Fig. 4 are from different perspectives, but no lipid bilayer derived from an exosome was observed in both images. This suggests that the shape and/or structure of the exosome collected by ultracentrifugation was disrupted.

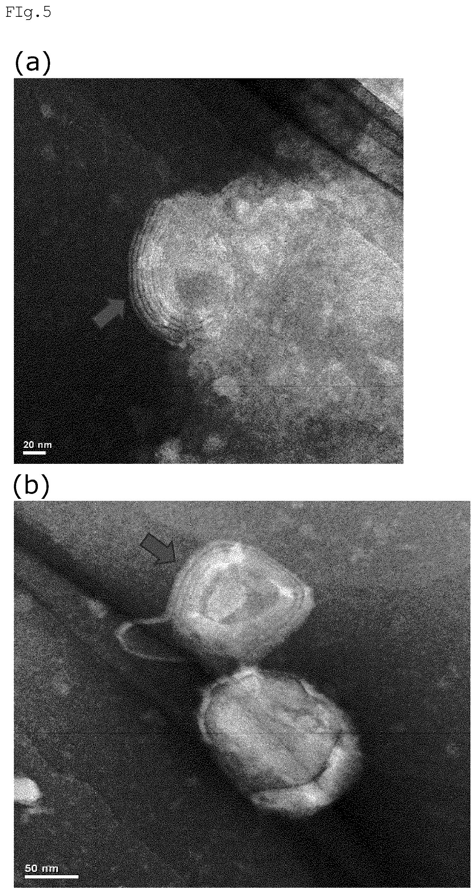

Fig. 5 is TEM images of exosomes collected using a conjugate prepared by binding a ligand molecule (anti-CD81) described later to the copolymer of Example 6. Lipid bilayers of exosomes are clearly observed in the images of (a) and (b) in Fig. 5. That is, it is suggested that exosomes were collected without disputing the shape and structure of the exosomes.

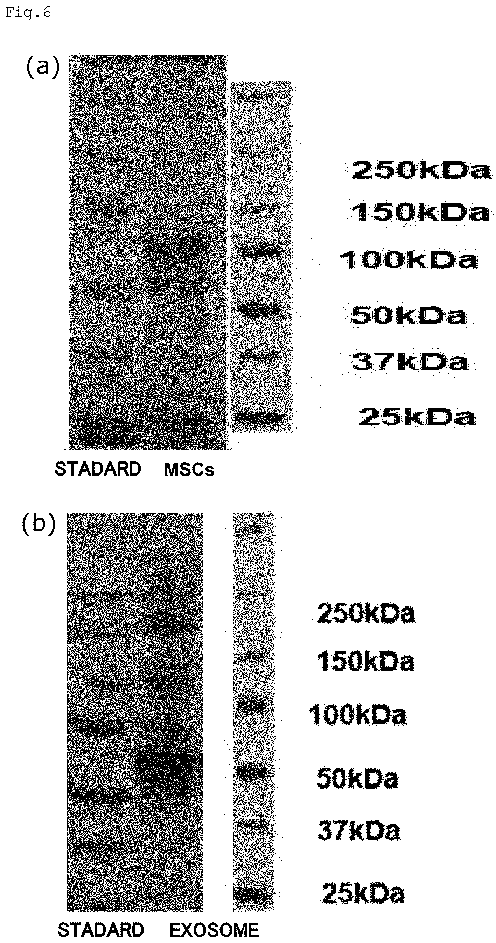

Fig. 6 is images showing the results of SDS-PAGE. Fig. 6(a) shows the results of MSCs, and Fig. 6(b) shows the results of collected exosomes. According to the above results, it is clarified that the collected exosomes were (lower molecular weight) components clearly different from MSCs.

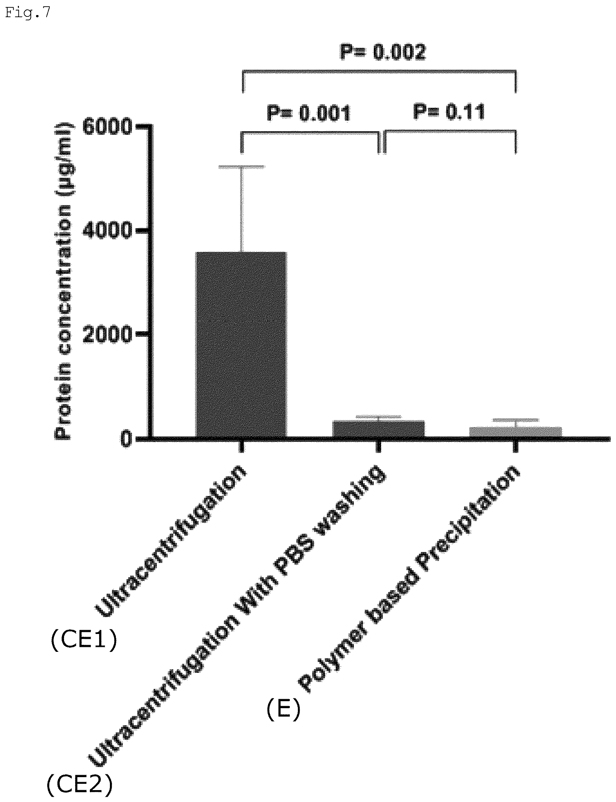

Fig. 7 is a graph showing the measurement results of protein concentrations with a microvolume spectrophotometer (NanoDrop): the exosome collected by ultracentrifugation (CE1), the exosome collected with ultracentrifugation and then washed with PBS (CE2), and the exosome collected by the method using the conjugate of Example 6 (E). According to the above results, it was demonstrated that the protein concentration in the exosome (E) collected by the method using the conjugate of Example 6 was lower than that in the exosome (CE1) collected by ultracentrifugation and was similar to that in the exosome (CE2) after washing with PBS. That is, it was suggested that contamination with protein can be suppressed by collecting the method using the conjugate of Example 6.

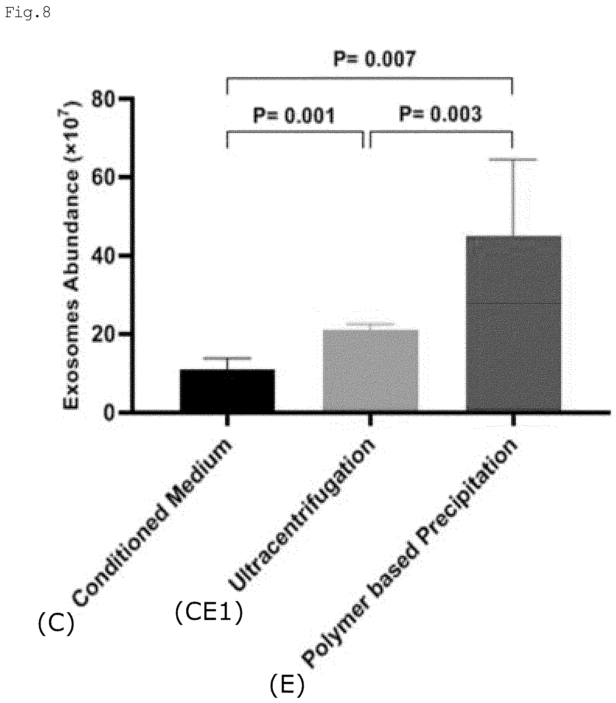

Fig. 8 is a graph showing the verification results of the amount of collected exosomes. The concentration of the exosome (E) collected using the conjugate of Example 6 greatly increased compared to the concentration in the medium (C) and also was significantly high compared to the exosome (CE1) collected by ultracentrifugation.

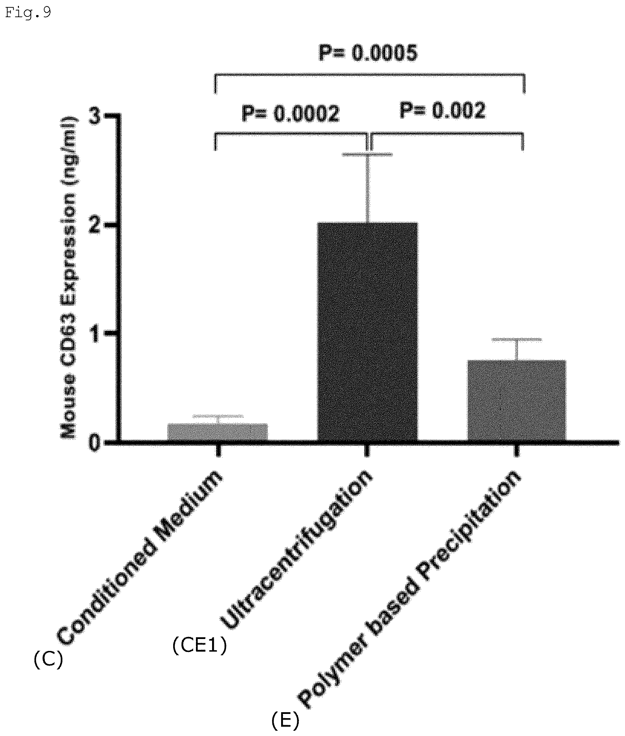

Fig. 9 is a graph showing the expression amounts of CD63 (surface marker of exosome) in collected exosomes. From the results, it was demonstrated that the expression amount of CD63 in the exosome (CE1) collected by ultracentrifugation was greatly higher than that in the medium (C) and that the increment amount in the exosome (E) collected by the method using the conjugate of Example 6 was suppressed. It is suggested that CD63 in a free state was increased in the exosome (CE1) collected by ultracentrifugation. In contrast, the reaction derived from CD63 in the exosome (E) collected using the conjugate of Example 6 was kept low. This also demonstrated that the shape and structure of the exosome were maintained in the exosome (E) collected using the conjugate of Example 6.

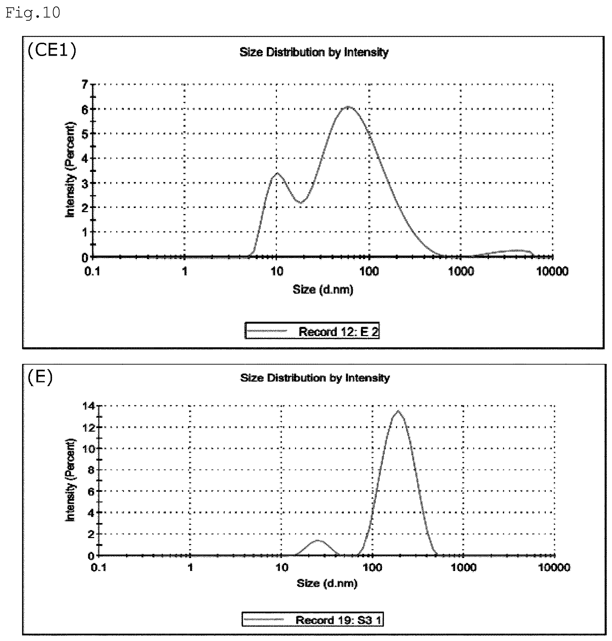

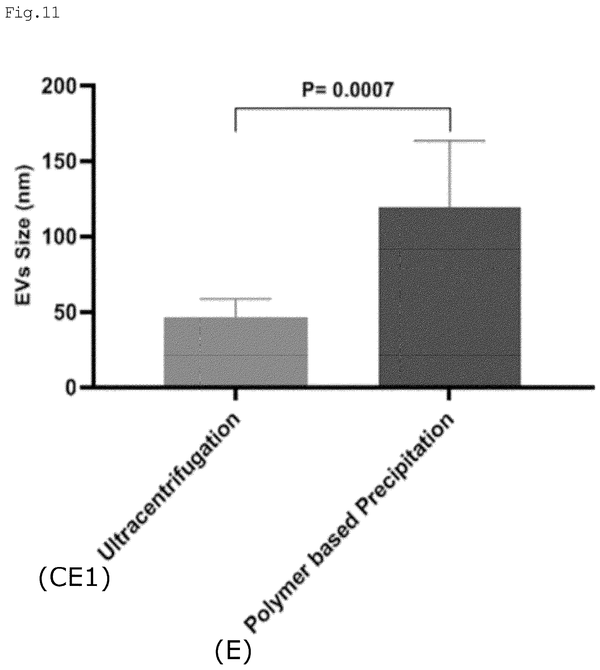

Fig. 10 is graphs showing the particle diameter measurement results of exosomes by DLS. The results of the exosome (CE1) collected using ultracentrifugation and the exosome (E) collected using the conjugate of Example 6 are shown. Fig. 11 is a graph showing the average diameters of the respective particles. According to Figs. 10 and 11, it is demonstrated that the exosome (E) collected using the conjugate of Example 6 has a larger particle diameter. In contract, in the exosome (CE1) collected by ultracentrifugation, the maximum diameter is also small and the distribution is large, which suggests occurrence of disruption of the exosomes.

From the above results, it was clarified that an exosome can be collected while maintaining the structure and shape of the exosome, compared to the conventional method by ultracentrifugation, by using the conjugate prepared by a copolymer of Example 6, a linker, and a ligand molecule (anti-CD81). It is not shown in the drawings, but it was confirmed that even when an anti-CD9 antibody is used as the ligand molecule, as in above, an exosome can be collected while maintaining the structure and shape.

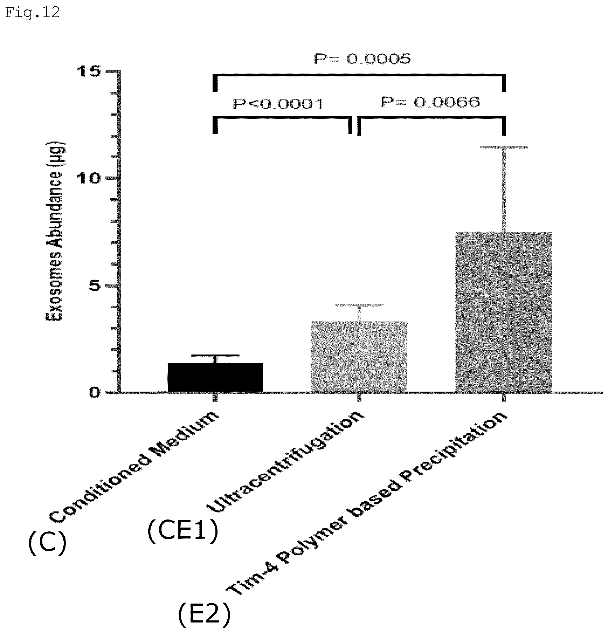

Fig. 12 is a graph showing the verification results of the amount of collected exosome when a conjugate prepared as in above except that Tim-4, which is a protein that binds to a phosphatidylserine group in a calcium ion-dependent manner, was used as a ligand molecule instead of the anti-CD81 antibody or the anti-CD9 antibody. The concentration of the exosome (E) collected using the conjugate of Example 6 was greatly increased compared to the concentration in the medium (C) and was also significantly high compared to the concentration of the exosome (CE1) collected by ultracentrifugation. The binding of Tim-4 and the copolymer of Example 6 was verified by SDS-PAGE as in above.

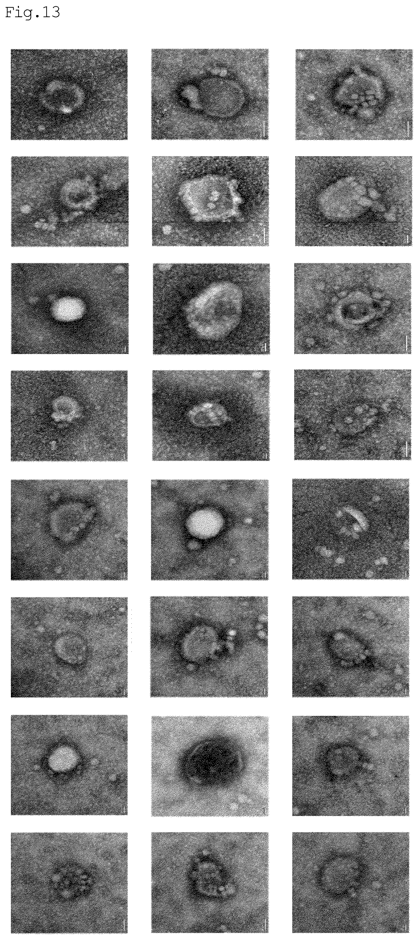

Fig. 13 is photographs showing the TEM observation results of exosomes separated by acting a chelating agent (EDTA) on a conjugate collected by heating and aggregation. In Fig. 13, images arranged in tiles are of exosomes detected from different perspectives, and it was confirmed that the shape and structure were maintained even after dissociation from the conjugate. It was also demonstrated that the particle diameter of the obtained exosome was the same as the results of the exosome (E) in Fig. 10 (average particle diameter: about 100 nm).

### <Relationship between composition of copolymer and exosome-concentrating efficiency>

The total amount of exosomes was quantitatively measured using "ExoQuant", FluoroCet kit, and Mouse CD63 ELISA Kit (Lifespan Bioscience Inc.) according to the instruction of the manufacturer.

An azide-modified TIM-4 protein (1 mg/mL, 100 µL in PBS) and a calcium chloride solution were added to a conditioned medium (5 mL) containing an exosome, followed by incubation at 37°C for 1 hour. Subsequently, copolymers with different SAKIPAAm ratios (protein : copolymer = 1 : 30) were subjected to click reaction at 4°C for 6 hours to be introduced into the azide-modified protein. The reaction mixture was centrifugated at 37°C at 13,800 ×g for 5 minutes, and the concentrated precipitate was then collected. The eluate containing Tris HCL and EDTA buffer solution was added to the collected concentrated product, and the mixture was stirred at 4°C for 1 hour. Finally, the solution was centrifugated at 37°C at 13,800 ×g for 5 minutes, and the released exosome was collected. The total protein amount including exosomes in the collected exosome solution was measured using Micro BCA Protein Assay Kit (Thermo Fisher Scientific).

Table 2 shows the results of the above test. As shown in Table 2, when the copolymer of Example 2 in which the content of SAKIPAAm (corresponding to unit 2) is 2.5 mol% or more was used, the exosome-concentrating efficiency was higher than when the copolymer (SAKIPAAm: 2.4 mol%) of Example 1 was used. When the copolymer of Example 6 in which the content of SAKIPAAm was 9.5 mol% or less was used, the exosome-concentrating efficiency was higher than when the copolymer (SAKIPAAm: 14 mol%) of Example 7 was used. In addition, as shown in Table 2, when the copolymer of Example 4 in which the content of SAKIPAAm (corresponding to unit 2) was 4.1 mol% or more was used, the exosome-concentrating efficiency was higher than when the copolymer (SAKIPAAm: 3.8 mol%) of Example 3 was used.

| Table 2 | SAKIPAAm content (mol%) | Concentrated amount of exosome (µg/mL) |
|---|---|---|
| Conditioned medium | | 6.4 |
| Example 1 | 2.4 | 29.4 |
| Example 2 | 2.5 | 31.2 |
| Example 3 | 3.8 | 33.9 |
| Example 4 | 4.1 | 34.2 |
| Example 5 | 6.0 | 35.8 |
| Example 6 | 9.5 | 40.0 |
| Example 7 | 14 | 25.8 |

## Claims

1. A copolymer comprising a unit 1 represented by a following formula 1 and a unit 2 represented by a following formula 2, wherein
when total units included in the copolymer is 100 mol%, the content of the unit 2 is 2.5 mol% or more and 9.5 mol% or less, 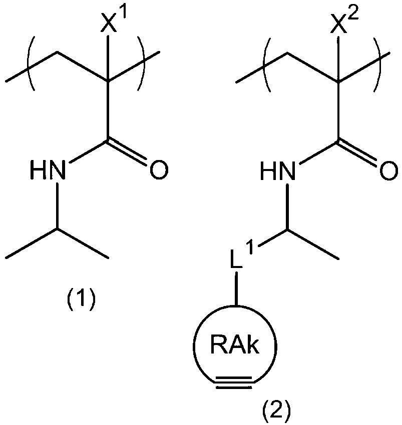 (in the formula 1, X¹ represents a hydrogen atom or a linear or branched alkyl group having 1 to 6 carbon atoms, and in the formula 2, X² represents a hydrogen atom or a linear or branched alkyl group having 1 to 6 carbon atoms, L¹ represents a divalent linking group, and RAk represents a cyclic alkynyl group having a monocyclic structure or a fused ring structure).

2. Use of the copolymer according to claim 1 in an exosome collection kit.

3. A conjugate preparation kit comprising the copolymer according to claim 1 or 2 and a linker represented by a following formula 4: 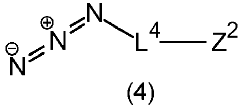 (in the formula 4, L⁴ represents a divalent linking group, and Z² represents a reactive group that can bind to a ligand molecule selected from the group consisting of a protein, an antibody, a peptide, and derivatives thereof).

4. The conjugate preparation kit according to claim 3, wherein the ligand molecule is a molecule targeting a marker that is present in outer membrane of an exosome.

5. The conjugate preparation kit according to claim 4, wherein the marker is a phosphatidylserine group.

6. The conjugate preparation kit according to claim 4 or 5, wherein the ligand molecule is a protein that binds to the marker metal-ion-dependently.

7. An exosome collection kit comprising the copolymer according to claim 1, a linker represented by a following formula 4, and a ligand molecule selected from the group consisting of a protein, an antibody, a peptide, and derivatives thereof, 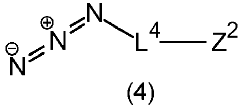 (in the formula 4, L⁴ represents a divalent linking group, and Z² represents a reactive group that can bind to the ligand molecule).

8. The exosome collection kit according to claim 7, wherein the ligand molecule is a molecule targeting a marker that is present in outer membrane of an exosome.

9. The exosome collection kit according to claim 8, wherein the marker is a phosphatidylserine group.

10. The exosome collection kit according to claim 8 or 9, wherein the ligand molecule is a protein that binds to the marker metal-ion-dependently.

11. A method for producing a conjugate for exosome collection, the method comprising:
forming a linker-ligand molecule complex by binding a linker represented by a following formula 4 and a ligand molecule selected from the group consisting of a protein, an antibody, a peptide, and derivatives thereof; and
generating a conjugate by reacting the linker-ligand molecule complex and a copolymer according to claim1, 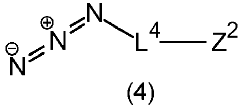 (in the formula 4, L⁴ represents a divalent linking group, and Z² represents a reactive group that can bind to the ligand molecule).

12. An exosome-collecting method comprising:
in the exosome collection kit of claim 7, forming a linker-ligand molecule complex by binding the linker and the ligand molecule;
forming a conjugate by reacting the linker-ligand molecule complex and the copolymer;
bringing the conjugate into contact with an exosome in a solution to immobilize at least a part of the exosome to the conjugate through the ligand molecule; and
heating the solution to a temperature not lower than an aggregation temperature set in advance based on the lower critical solution temperature of the copolymer to aggregate the exosome together with the conjugate.

13. The exosome-collecting method according to claim 12, wherein the ligand molecule is a molecule targeting a marker that is present in outer membrane of an exosome.

14. The exosome-collecting method according to claim 13, wherein the marker is a phosphatidylserine group.

15. The exosome-collecting method according to claim 13 or 14, wherein the ligand molecule is a protein that binds to the marker metal-ion-dependently.
